# EUROPÄISCHE PATENTSCHRIFT

(11) **EP 0 646 590 B1**
(45) Veröffentlichungstag und Bekanntmachung des Hinweises auf die Patenterteilung: **25.08.1999**
(21) Anmeldenummer: 94810548.1
(22) Anmeldetag: 22.09.1994
(51) Int. Cl.: C07F 17/02, B01J 31/28, C07B 31/00, C07B 53/00

(54) **Mit Fluoralkyl substituierte Ferrocenyldiphosphine als Liganden für homogene Katalysatoren**
Ferrocenyldiphosphines substituted with fluoroalkyl groups as ligands for homogeneous catalysts
Ferrocényldiphosphines substituées par un groupe fluoroalkyle comme ligands pour des catalyseurs homogènes

(30) Priorität: 01.10.1993 CH 297293
(43) Veröffentlichungstag der Anmeldung: 05.04.1995
(73) Patentinhaber: Novartis AG, 4058 Basel (CH)
(72) Erfinder: Spindler, Felix, Dr., CH-4656 Starrkirch-Wil (CH)

(56) Entgegenhaltungen:
- EP-A- 0 564 406
- EP-A- 0 612 758
- US-A- 5 245 064
- BULLETIN OF THE CHEMICAL SOCIETY OF JAPAN, Bd.53, Nr.4, 1980, TOKYO JP Seiten 1138 - 1151 T. HAYASHI 'Asymmetric synthesis catalized by chiral ferrocenylphosphine-transition metal complexes.'

## Beschreibung

Die Erfindung betrifft 1-[2-Phosphinoferrocenyl]alkylidenphosphine in Form von Racematen und Stereoisomeren, ein Verfahren zu deren Herstellung, lridium- und Rhodium-Komplexe mit diesen Liganden und deren Verwendung als enantioselektive Hydrierkatalysatoren für die homogene Hydrierung von prochiralen ungesättigten Verbindungen.

T. Hayashi et al. beschreiben im Bull. Chem. Soc. Jpn., 53, Seiten 1136-1151 die Herstellung von einem chiralen Ferrocenylphosphin als Ligand für Übergangsmetallkomplexe zur asymmetrischen Synthese, nämlich [(R)-[(S)-2-(Diphenylphosphino)ferrocenyl]ethyl]diphenylphosphin. Anwendungen sind für diese Verbindungen nicht beschrieben. In der nachpublizierten EP-A-0 564 406 ist die Verbindung {(R)-1-[(S)-2-(Diphenylphosphino)ferrocenyl]}ethyl-di-(4-trifluromethylphenyl)phosphin beschrieben.

Es wurde nun gefunden, dass Fluoralkyl substituierte 1-[2-Phosphinoferrocenyl]-alkylidenphosphine wirksame Katalysatoren für die asymmetrische Hydrierung sind, und dass bei kurzen Reaktionszeiten eine hohe Enantioselektivität erreicht werden kann. Ein weiterer Vorteil dieser Verbindungen ist, dass die daraus hergestellten Rhodium-und besonders Iridium-Komplexe nur eine geringe Tendenz zur Desaktivierung aufweisen, so dass Hydrierungen bei höheren Temperaturen durchgeführt werden können, was eine grössere Katalysatorproduktivität zur Folge hat.

Ein Gegenstand der Erfindung sind Verbindungen der Formel I, 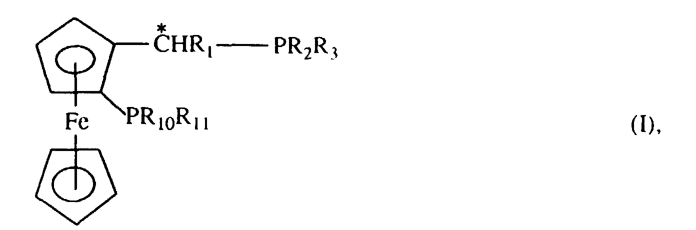 worin R₁ C₁-C₈-Alkyl, Phenyl oder mit 1 bis 3 C₁-C₄-Alkyl oder C₁-C₄-Alkoxy substituiertes Phenyl bedeutet; R₂ einen Rest der Formel II 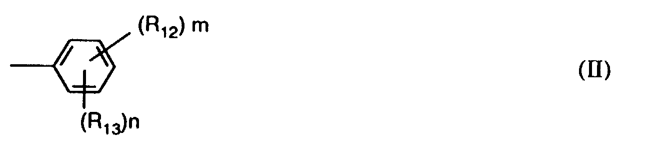 darstellt, worin R₁₂ teilweise oder vollständig mit Fluor substituiertes C₁-C₅-Alkyl bedeutet;
R₁₃ C₁-C₄-Alkyl, C₁-C₄-Alkoxy, -SiR₄R₅R₆, Halogen, -SO₃M, -CO₂M, -PO₃M, -NR₇R₈ oder -[^{⊕}NR₇R₈R₉]X^{⊖} ist;
m eine Zahl von 1 bis 3 ist, n für 0 oder eine Zahl von 1 bis 4 steht und die Summe von m+n 1 bis 5 ist;
R₃, R₁₀, R₁₁ unabhängig voneinander die gleiche Bedeutung wie R₂ haben oder unabhängig voneinander C₁-C₁₂-Alkyl, C₅-C₁₂-Cycloalkyl, Phenyl, mit C₁-C₄-Alkyl oder C₁-C₄-Alkoxy substituiertes C₅-C₁₂-Cycloalkyl oder mit ein bis drei C₁-C₄-Alkyl, C₁-C₄-Alkoxy, -SiR₄R₅R₆, Halogen, -SO₃M, -CO₂M, -PO₃M, -NR₇R₈ oder -[^{⊕}NR₇R₈R₉]X^{⊖} substituiertes Phenyl darstellen;
R₄, R₅ und R₆ unabhängig voneinander für C₁-C₁₂-Alkyl oder Phenyl stehen;
R₇ und R₈ H, C₁-C₁₂-Alkyl, Phenyl oder
R₇ und R₈ zusammen Tetramethylen, Pentamethylen oder 3-Oxa-1,5-Pentylen darstellen;
R₉ H oder C₁-C₄-Alkyl bedeutet,
M für H oder ein Alkalimetall steht, X^{⊖} das Anion einer einbasischen Säure ist, und * ein stereogenes C-Atom kennzeichnet, in Form ihrer Racemate und Diastereomeren oder Gemischen von Diastereomeren, mit Ausnahme von {(R)-1-[(S)-2-(Diphenylphosphino)ferrocenyl]}ethyl-di-(4-trifluoromethylphenyl)phosphin.

In Formel II stehen m bevorzugt für 1 oder 2 und n für 0, 1 oder 2. Insbesondere steht n für 0.

R₁ in der Bedeutung von Alkyl ist vorzugsweise linear. Es enthält bevorzugt 1 bis 4 C-Atome. Beispiele für dieses Alkyl sind Methyl, Ethyl, n- und i-Propyl, n-, i- und t-Butyl, Pentyl, Hexyl, Heptyl und Octyl. Bevorzugt sind Methyl und Ethyl und besonders bevorzugt ist Methyl.

R₁ enthält als substituiertes Phenyl bevorzugt 1 oder 2 Substituenten. Alkylsubstituenten können zum Beispiel Methyl, Ethyl, n- und i-Propyl, n-, i- und t-Butyl sein; bevorzugt sind Methyl und Ethyl. Alkoxysubstituenten können zum Beispiel Methoxy, Ethoxy, n-und i-Propoxy, n-, i- und t-Butoxy sein; bevorzugt sind Methoxy und Ethoxy. In einer Gruppe von Verbindungen der Formel I steht R₁ vorzugsweise für Phenyl oder mit 1 oder 2 C₁-C₄-Alkyl oder C₁-C₄-Alkoxy substituiertes Phenyl.

R₃, R₁₀ und R₁₁ in der Bedeutung von Alkyl können linear oder verzweigt sein und sie enthalten bevorzugt 1 bis 8 und besonders bevorzugt 1 bis 4 C-Atome. Beispiele für dieses Alkyl sind Methyl, Ethyl, n- und i-Propyl, n-, i- und t-Butyl, Pentyl, Hexyl, Heptyl, Octyl, Nonyl, Decyl, Undecyl und Dodecyl. Bevorzugt sind Methyl, Ethyl, n- und i-Propyl, n-, i- und t-Butyl. Wenn R₃ und/oder R₁₀ und R₁₁ gleich sind, bedeuten sie als Alkyl besonders bevorzugt i-Propyl oder t-Butyl.

R₃, R₁₀ und R₁₁ in der Bedeutung von Cycloalkyl enthalten bevorzugt 5 bis 8, besonders bevorzugt 5 oder 6 Ring-C-Atome. Beispiele für Cycloalkyl sind Cyclopentyl, Cyclohexyl, Cycloheptyl, Cyclooctyl, Cyclodecyl und Cyclododecyl. Bevorzugt sind Cyclopentyl und Cyclohexyl und besonders bevorzugt ist Cyclohexyl.

Das Cycloalkyl kann substituiert sein, zum Beispiel mit 1 bis 3 Alkyl- oder Alkoxy-Substituenten, Beispiele für solche Substituenten sind zuvor angegeben worden. Bevorzugt sind Methyl und Ethyl sowie Methoxy und Ethoxy. Beispiele für substituiertes Cycloalkyl sind Methyl- und Methoxycyclopentyl und -cyclohexyl.

R₃, R₁₀ und R₁₁ in der Bedeutung von substituiertem Phenyl enthält bevorzugt 1 oder 2 Substituenten. Sofern das Phenyl 2 oder 3 Substituenten enthält, können diese gleich oder verschieden sein.

Beispiele für die Substituenten Alkyl und Alkoxy sind zuvor angegeben worden; bevorzugte Alkyl- und Alkoxysubstituenten für Phenyl sind Methyl, Ethyl sowie Methoxy und Ethoxy.

Wenn der Phenyl-Substituent Halogen bedeutet, so handelt es sich bevorzugt um -F, -Cl und -Br.

R₄, R₅ und R₆ können lineares oder verzweigtes Alkyl sein, das bevorzugt 1 bis 8 und besonders bevorzugt 1 bis 4 C-Atome enthält. Beispiele für Alkyl sind zuvor angegeben worden. Bevorzugtes Alkyl ist Methyl, Ethyl, n-Propyl, n-Butyl und t-Butyl. Besonders bevorzugt steht der Substituent -SiR₄R₅R₆ für Trimethylsilyl.

Unter den sauren Phenyl-Substituenten -SO₃M, -CO₂M und -PO₃M ist die Gruppe -SO₃M bevorzugt. M steht bevorzugt für H, Li, Na und K.

R₇ und R₈ enthalten als Alkyl bevorzugt 1 bis 6, besonders bevorzugt 1 bis 4 C-Atome. Das Alkyl ist bevorzugt linear. Bevorzugte Beispiele sind Methyl, Ethyl, n-Propyl und n-Butyl. R₉ stellt als Alkyl bevorzugt Methyl dar.

X^{⊖} stellt als Anion einer einbasischen Säure bevorzugt Cl^{⊖}, Br^{⊖} oder das Anion einer Carbonsäure dar, zum Beispiel Formiat, Acetat, Trichloracetat oder Trifluoracetat dar.

Einige Beispiele für substituiertes Phenyl sind 2-Methyl-, 3-Methyl-, 4-Methyl-, 2- oder 4-Ethyl-, 2- oder 4-i-Propyl-, 2- oder 4-t-Butyl-, 2-Methoxy-, 3-Methoxy-, 4-Methoxy-, 2- oder 4-Ethoxy-, 4-Trimethylsilyl-, 2- oder 4-Fluor-, 2,4-Difluor-, 2- oder 4-Chlor-, 2,4-Dichlor-, 2,4-Dimethyl-, 3,5-Dimethyl-, 2-Methoxy-4-methyl-, 3,5-Dimethyl-4-methoxy-, 3,5-Dimethyl-4-(dimethylamino)-, 2- oder 4-Amino-, 2- oder 4-Methylamino-, 2- oder 4-(Dimethylamnio)-, 2- oder 4-SO₃H-, 2- oder 4-SO₃Na-, 2- oder 4-[^{⊕}NH₃Cl^{⊖}]-, 3,4,5-Trimethylphen-1-yl oder 2,4,6-Trimethylphen-1-yl.

R₁₂ bedeutet bevorzugt teilweise oder vollständig mit Fluor substituiertes C₁-C₃-Alkyl und besonders bevorzugt C₁-oder C₂-Alkyl. Beispiele für teilweise fluoriertes C₁-C₅-Alkyl sind die Stellungsisomeren von Mono- bis Decafluorpentyl, Mono- bis Octafluorbutyl, Mono- bis Hexafluorpropyl, Mono- bis Tetrafluorethyl sowie Mono- und Difluormethyl. Unter den teilweise fluoriererten Alkylresten sind solche der Formeln -CF₂H und -CF₂(C₁-C₄-Alkyl) besonders bevorzugt. Besonders bevorzugt bedeutet R₁₂ ein perfluoriertes Alkyl. Beispiele hierfür sind Perfluorpentyl, Perfluorbutyl, Perfluorpropyl, Perfluorethyl und insbesondere Trifluormethyl. Die mit Fluor substituierten Alkylgruppen sind bevorzugt in den 3-, 4- und 5- Stellungen gebunden.

In einer besonders bevorzugten Form ist R₂ 4-Trifluormethyl-phenyl oder 3, 5-Di-(trifluormethyl)-phenyl.

Wenn n für 1 steht, bedeutet R₁₃ bevorzugt C₁-C₄-Alkyl oder C₁-C₄- Alkoxy. Bevorzugt steht n für 0. Beispiele für bevorzugte Alkyl und Alkoxy sind vorstehend angegeben.

In einer bevorzugten Ausführungsform stellen R₃, R₁₀ und R₁₁ gleiche Reste dar und bedeuten Phenyl, Cyclohexyl, t-Butyl, 2- oder 4-Methylphen-1-yl, 2- oder 4-Methoxyphen-1-yl, 2- oder 4-(Dimethylamino)phen-1-yl, 3,5-Dimethyl-4-(dimethylamino)phen-1-yl oder 3,5-Dimethyl-4-methoxyphen-1-yl bedeuten.

Besonders bevorzugt sind R₂ und R₃ gleich und stellen einen Rest der Formel II dar, wobei R₁₀ und R₁₁ bevorzugt gleiche Reste darstellen und Phenyl, Cyclohexyl, t-Butyl, 2- oder 4-Methylphen-1-yl, 2- oder 4-Methoxyphen-1-yl, 2- oder 4-(Dimethylamino)phen-1-yl, 3,5-Dimethyl-4-(dimethylamino)phen-1-yl oder 3,5-Dimethyl-4-methoxyphen-1-yl bedeuten.

In einer weiteren bevorzugten Ausführungsform sind R₂ und R₃ gleich und stellen einen Rest der Formel II dar, R₁₀ steht für Phenyl und R₁₁ bedeutet Cyclohexyl, 2- oder 4-Methylphen-1-yl, 2- oder 4-Methoxyphen-1-yl, 4-(Dimethylamino)phen-1-yl, 3,5-Dimethyl-4-(dimethylamino)phen-1-yl, 3,5-Dimethyl-4-methoxyphen-1-yl oder 4-t-Butyl-phen-1-yl.

Wenn R₂ und R₃ gleich sind und den in der Formel II angegebenen Rest darstellen, sind R₁ besonders bevorzugt Methyl und R₁₀ und R₁₁ Phenyl oder Cyclohexyl.

In einer anderen bevorzugten Ausführungsform sind R₂, R₃, R₁₀ und R₁₁ gleich und stellen einen Rest der Formel II dar.

Ganz besonders bevorzugt sind Verbindungen der Formel I, worin R₁ Methyl ist, R₂, R₃, R₁₀ und R₁₁ gleich sind und einen Rest der Formel II darstellen;
R₁₂ 4-Trifluormethyl oder 3,5-Di(trifluormethyl) bedeutet und n 0 ist. In einer anderen besonders bevorzugten Ausführungsform ist R₁ Methyl, R₂ und R₃ sind gleich und bedeuten einen in der Formel II angegebene Rest, R₁₀ und R₁₁ sind Phenyl, R₁₂ bedeutet 4-Trifluormethyl oder 3,5-Di(trifluormethyl) und n ist 0.

Als ganz besonders bevorzugte Verbindungen der Formel I sind zu nennen:
{(R)-1-[(S)-2-(Diphenylphosphino)ferrocenyl]}ethyl-di(4-trifluoromethylphenyl)phosphin,
{(R)-1-[(S)-2-(Diphenylphosphino)ferrocenyl]}ethyl-di(3,5-bis-(trifluoromethyl)-phenyl)phosphin,
{(R)-1-[(S)-2-(Di-(4-trifluoromethylphenyl)-phosphino)ferrocenyl]}ethyl-di(4-trifluoromethylphenyl)phosphin,
{(R)-1-[(S)-2-(Di-(4-trifluoromethylphenyl)-phosphino)ferrocenyl]}ethyl-di(3,5-dimethylphenyl)phosphin,
{(R)-1-[(S)-2-(Di-(4-trifluoromethylphenyl)-phosphino)ferrocenyl]}ethyl-dicyclo-hexylphosphin,
{(R)-1-[(S)-2-(Di-(3,5-bis-(trifluoromethyl)phenyl)phosphino)ferrocenyl]}ethyl-di(3,5-dimethylphenyl)phosphin,
{(R)-1-[(S)-2-(Di-(3,5-bis-(trifluoromethyl)phenyl)phosphino)ferrocenyl]}ethyl-diphenylphosphin,
{(R)-1-[(S)-2-(Di-(4-trifluoromethylphenyl)phosphino)ferrocenyl]}ethyl-diphenylphosphin.

Die Verbindungen der Formel I können hergestellt werden, indem man entweder eine Verbindung der Formel III 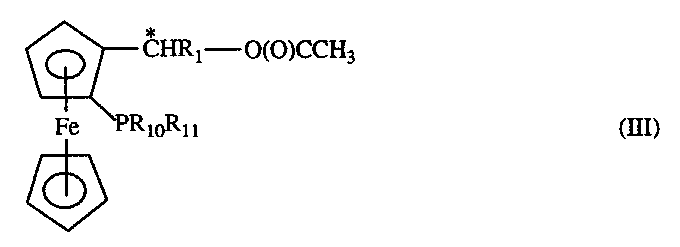 in Gegenwart eines inerten Lösungsmittels bei Raumtemperatur oder erhöhter Temperatur mit einem Phosphin der Formel IV

HPR₂R₃ (IV)

umsetzt; oder eine Verbindung der Formel VII 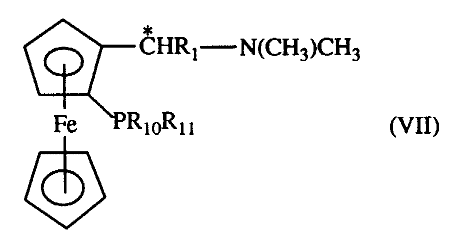 worin R₁, R₁₀ und R₁₁ die unter Formel I angegebenen Bedeutungen haben, in Gegenwart eines inerten Lösungsmittels bei Raumtemperatur oder erhöhter Temperatur mit einem Phosphin der Formel IV

HPR₂R₃ (IV)

umsetzt. Dieses Verfahren bildet einen weiteren Gegenstand der vorliegenden Erfindung.

Die Umsetzungen sind an sich bekannt und von T. Hayashi et al. im Bull. Chem. Soc. Jpn., 53, Seiten 1136-1151 beschrieben. Die Herstellung von Verbindungen der Formel III und VII ist dort ebenfalls beschrieben oder kann analog erfolgen. Die Phosphine der Formel IV sind bekannt oder nach bekannten Verfahren in analoger Weise herstellbar.

Die Reaktionstemperatur kann zum Beispiel 20 bis 150 °C, bevorzugt 40 bis 100 °C betragen. Als Lösungsmittel eignen sich polare protische und aprotische Lösungsmittel, die alleine oder in Mischung von zwei oder mehreren Lösungsmitteln verwendet werden können. Einige Beispiele für Lösungsmittel sind Alkanole wie zum Beispiel Methanol und Ethanol, und Carbonsäuren wie zum Beispiel Ameisensäure und Essigsäure.

Die Verbindungen der Formel I werden als Racemate, Gemische von Stereoisomeren oder als Stereoisomere erhalten, je nach dem, ob die Verbindungen der Formel III als Racemate, Gemische von Stereoisomeren oder als Stereoisomere eingesetzt werden. Racemate und Gemische von Stereoisomeren können mittels bekannter Methoden in die Stereoisomeren getrennt werden, wobei im allgemeinen chromatographische Methoden bevorzugt werden.

Die Isolierung und Reinigung der Verbindungen der Formel I erfolgt nach an sich bekannten Methoden, zum Beispiel Destillation, Extraktion, Kristallisation und/oder chromatographischen Methoden.

Die erfindungsgemässen Verbindungen der Formel I eignen sich als Liganden für Rhodium- und Iridiumkomplexe. Ein weiterer Gegenstand der Erfindung sind Komplexe der Formeln V und VI,

[X₁M₁YZ] (V),

[X₁M₁Y]^{⊕}A₁^{⊖} (VI),

worin X₁ für zwei C₂-C₁₂-Olefine oder ein C₅-C₁₂-Dien steht, Z für Cl, Br oder I steht, A₁^{⊖} das Anion einer Sauerstoffsäure oder Komplexsäure bedeutet, M₁ für Rh oder Ir steht und Y ein erfindungsgemässes Diphosphin der Formel I darstellt. Die Komplexe der Formel VI sind bevorzugt.

Für die Diphosphine der Formel I gelten die gleichen zuvor angeführten Bevorzugungen und beispielhaften Ausführungen. X₁ in der Bedeutung als Olefin enthält bevorzugt 2 bis 6 und besonders bevorzugt 2 bis 4 C-Atome. Besonders bevorzugt ist Ethylen; weitere Beispiele sind Propen und 1-Buten. X₁ enthält als Dien bevorzugt 5 bis 8 C-Atome, wobei es sich um ein offenkettiges oder mono- oder bicyclisches Dien handeln kann. Die beiden Olefingruppen des Diens sind bevorzugt durch ein oder zwei CH₂-Gruppen verbunden. Beispiele sind 1,3-Pentadien, Cyclopentadien, 1,5-Hexadien, 1,4-Cyclohexadien, 1,4- oder 1,5-Heptadien, 1,4- oder 1,5-Cycloheptadien, 1,4- oder 1,5-Octadien, 1,4- oder 1,5-Cyclooctadien, Norbornadien. Bevorzugt stellt X₁ zwei Ethylen, 1,5-Hexadien, 1,5-Cyclooctadien oder Norbornadien dar.

In Formel V steht Z bevorzugt für Cl oder Br. Beispiele für A₁^{⊖} in Formel VI sind ClO₄^{⊖}, FSO₃^{⊖}, CH₃SO₃^{⊖}, CF₃SO₃^{⊖}, BF₄^{⊖} , PF₆^{⊖}, SbCl₆^{⊖} , AsF₆^{⊖} und SbF₆^{⊖}. Bevorzugt ist A₁^{⊖} ClO₄^{⊖}, CF₃SO₃^{⊖}, BF₄^{⊖}, PF₆^{⊖} und SbF₆^{⊖}.

Die erfindungsgemässen Komplexe werden in an sich bekannter Weise durch die Umsetzung von äquimolaren Mengen einer Verbindung der Formel I mit einem Metallkomplex der Formeln [M₁(X₁)Z]₂ oder M₁(X₁)₂^{⊕} A₁^{⊖} erhalten, worin M₁, X₁, Z und A₁^{⊖} die zuvor angegebenen Bedeutungen haben. Die Metallkomplexe sind bekannt, siehe zum Beispiel EP-A-0 302 021 uns US-A-5 011 995.

Die Umsetzung wird vorteilhaft unter Inertgasatmosphäre, zum Beispiel Argon, und zweckmässig bei Temperaturen von 0 bis 40 °C, bevorzugt bei Raumtemperatur vorgenommen. Vorteilhaft wird ein Lösungsmittel oder Gemisch von Lösungsmitteln mitverwendet, zum Beispiel Kohlenwasserstoffe (Benzol, Toluol, Xylol), halogenierte Kohlenwasserstoffe (Methylenchlorid, Chloroform, Chlorbenzol), Alkanole (Methanol, Ethanol, Ethylenglykolmonomethylether) und Ether (Diethylether, Dibutylether, Ethylenglykoldimethylether, Tetrahydrofuran, Dioxan) oder Gemische davon. Die erfindungsgemässen Komplexe können nach üblichen Methoden isoliert und gereinigt werden, oder sie können vor einer Hydrierung in situ hergestellt und dann in gelöster Form direkt als Hydrierkatalysator eingesetzt werden.

Die erfindungsgemässen Komplexe eignen sich hervorragend als homogene Katalysatoren zur enantioselektiven Hydrierung von prochiralen Verbindungen mit Kohlenstoff- und Kohlenstoff-/Heteroatomdoppelbindungen, zum Beispiel Verbindungen, die eine der Gruppen C=C, C=N, C=O, C=C-N oder C=C-O enthalten [siehe zum Beispiel K. E. König, The Applicability of Asymmetric Homogeneous Catalysis, in James D. Morrison (ed.), Asymmetric Synthesis, Vol. 5, Academic Press, 1985]. Beispiele für solche Verbindungen sind prochirale Olefine, Enamine, Imine und Ketone. Es werden überraschend hohe Ausbeuten, im allgemeinen sogar ein quantitativer chemischer Umsatz, in kurzen Reaktionszeiten erzielt. Besonders überraschend sind die sehr hohen optischen Ausbeuten, die mit den erfindungsgemässen Komplexen erreicht werden. Der Enantiomerenüberschuss (ee) kann über 90 % betragen. Es können hierbei Racemate, Gemische von Stereoisomeren oder Stereoisomere der Komplexe der Formeln V und VI verwendet werden, wobei Gemische von Stereoisomeren oder Stereoisomere bevorzugt sind.

Ein weiterer Gegenstand der Erfindung ist die Verwendung der erfindungsgemässen Komplexe der Formeln V und VI als homogene Katalysatoren zur asymmetrischen Hydrierung von prochiralen Verbindungen mit Kohlenstoff- oder Kohlenstoff-/Heteroatomdoppelbindungen, besonders mit Gruppen C=C, C=N, C=O, C=C-N oder C=C-O. Bevorzugt ist die Verwendung zur Hydrierung von unsymmetrischen Kohlenstoffdoppelbindungen, Ketiminen und Ketonen. Der erfindungsgemäss als Iridium-Komplex ausgebildete Katalysator der Formeln V und VI wird auch bevorzugt zur Hydrierung von prochiralen N-Arylketiminen zu optisch aktiven sekundären Aminen verwendet. Der erfindungsgemäss als Rhodium-Komplex ausgebildete Katalysator der Formeln V und VI wird bevorzugt zur Hydrierung von Kohlenstoffdoppelbindungen, zum Beispiel prochiralen Kohlenstoffdoppelbindungen verwendet.

Ein anderer Gegenstand der Erfindung ist ein Verfahren zur asymmetrischen Hydrierung von prochiralen Verbindungen mit Kohlenstoff- oder Kohlenstoff-/Heteroatomdoppelbindungen unter homogenen Reaktionsbedingungen, das dadurch gekennzeichnet ist, dass man die Verbindungen bei einer Temperatur von -20 bis 80 °C und einem Wasserstoffdruck von 10⁴ bis 10⁷ Pa in Gegenwart katalytischer Mengen eines Komplexes der Formeln V oder VI hydriert.

Bevorzugte prochirale Verbindungen sind zuvor erwähnt worden. Unsymmetrische Ketimine und Ketone sind bekannt. In Frage kommende N-Arylketimine sind zum Beispiel in der EP-A-0 256 982 beschrieben. N-aliphatische Ketimine sind zum Beispiel in der EP-A-0 301 457 beschrieben. Solche Imine können aus den entsprechenden unsymmetrische Ketonen hergestellt werden, die bekannt und teilweise käuflich oder nach bekannten Verfahren herstellbar sind. Geeignete substituierte Alkene sind in der zuvor erwähnten Publikation von K. E. König beschrieben.

Das Verfahren wird bevorzugt bei einer Temperatur von -10 bis 50 °C und bevorzugt bei einem Wasserstoffdruck von 1·10⁵ bis 6·10⁶ Pa durchgeführt.

Die Menge des Katalysators wird bevorzugt so gewählt, dass das Molverhältnis der zu hydrierenden Verbindung (Substrat) zu dem Komplex der Formeln V oder VI bevorzugt 100000 bis 20, besonders bevorzugt 20000 bis 40 und ganz besonders bevorzugt 1000 bis 50 beträgt.

Eine bevorzugte Verfahrensdurchführung ist dadurch gekennzeichnet, dass man zusätzlich ein Ammonium- oder Alkalimetallchlorid, -bromid oder -iodid mitverwendet, besonders bei der Verwendung erfindungsgemässer Iridium-Katalysatoren. Die Menge kann beispielsweise 0,1 bis 100, bevorzugt 1 bis 50 und besonders bevorzugt 2 bis 20 Aequivalente betragen, bezogen auf den Komplex der Formeln V oder VI. Der Zusatz von Iodiden ist bevorzugt. Ammonium ist bevorzugt Tetraalkylammonium mit 1 bis 6 C-Atomen in den Alkylgruppen, und als Alkalimetall ist Lithium, Natrium und Kalium bevorzugt.

Die Hydrierung kann ohne oder in Gegenwart von Lösungsmitteln durchgeführt werden. Geeignete Lösungsmittel, die alleine oder als Mischung von Lösungsmitteln eingesetzt werden können, sind zum Beispiel: Aliphatische und aromatische Kohlenwasserstoffe (Pentan, Hexan, Cyclohexan, Methylcyclohexan, Benzol, Toluol, Xylol), Alkohole wie z.B. Methanol, Ethanol, Propanol und Butanol; Ether wie z.B. Diethylether, Diethylenglykoldimethylether, Ethylenglykoldimethylether, Tetrahydrofuran und Dioxan; Halogenkohlenwasserstoffe wie z.B. Methylenchlorid, Chloroform, 1,1,2,2-Tetrachlorethan und Chlorbenzol; Ester und Lactone wie z.B. Essigsäureethylester, Butyrolacton oder Valerolacton; Carbonsäureamide und Lactame wie z.B. Dimethylformamid, Dimethylacetamid und N-Methylpyrrolidon. Bevorzugte Mischungen sind solche aus Alkoholen und aromatischen Kohlenwasserstoffen, zum Beispiel Methanol/Benzol oder Methanol/Toluol. Bevorzugt wird als Lösungsmittel Methanol alleine oder im Gemisch mit Benzol oder Toluol verwendet.

Mit dem erfindungsgemässen Hydrierverfahren können optisch reine Verbindungen erhalten werden, die wertvolle Zwischenprodukte zur Herstellung biologisch aktiver Wirkstoffe besonders im Bereich der Pharmazeutika und Agrarchemikalien darstellen. So können zum Beispiel aus sekundären Aminen, besonders N-Carbalkoxymethylaminen, 5-Imidazolcarbonsäurederivate hergestellt werden, die eine herbizide Wirkung haben und zur Bekämpfung von Unkräutern verwendet werden können (EP-A-0 207 563). Die optisch reinen α-Aminocarbonsäureester sind für Peptidsynthesen geeignet. Aus ungesättigten Aminocarbonsäuren können optisch reine Aminocarbonsäuren erhalten werden, die wertvolle Synthesebausteine darstellen.

Die nachfolgenden Beispiele erläutern die Erfindung näher. Die Reaktionen werden unter Argonatmosphäre durchgeführt. Der chemische Umsatz wird gaschromatographisch bestimmt [Säule DB 17/30 W (15 m), Hersteller: JCW Scientific INC., USA, Temperaturprogramm: 60°C/ 1 min bis 220°C, ΔT: 10°·min⁻¹]. Die Bestimmung der optischen Ausbeute Enantiomerenüberschuss, ee) erfolgt gaschromatographisch [Säulen Chirasil-Val (50 m), FS-Lipodex (50 m)] oder mittels HPLC (stationäre Phase: Chiralcel OD).

### Herstellungsbeispiele

### Beispiel A1: {(R)-1-[(S)-2-Diphenylphosphino)ferrocenyl]}ethyl-di(4-trifluoromethylphenyl)phosphin (A). (Liegt außerhalb des beanspruchten Bereiches).

In einen 50 ml-Schlenk-Kolben werden unter Argon-Atmosphäre nacheinander 1,2 g (2,73 mmol) N-{(R)-1-[(S)-diphenylphosphino)ferrocenyl]}ethyl-dimethylamin [hergestellt gemäss Bull. Chem. Soc. Jpn., **53**, 1138 (1980)], 25 ml Essigsäure und 1 g (3,1 mmol) Di(4-trifluoromethylphenyl)phosphin eingetragen und anschliessend während 60 Minuten unter Rühren bei 100°C erhitzt. Dann wird das Rohprodukt aus Wasser/Toluol extrahiert. Die organische Phase wird mit Magnesiumsulfat getrocknet und das Lösungsmittel am Rotationsverdampfer entfernt. Nach einer chromatographischen Reinigung über Kieselgel (Lösungsmitttel: Hexan/Dichlormethan (4:1)) und der anschliessenden Umkristallisation des Rohproduktes aus heissem Ethanol erhält man 1,49 g **A** (Ausbeute 76%) als orangegelbe kristalline Substanz.
³¹P-NMR (CDCl₃): -26,4 (d, J=24), 6,6 (d, J=24).

### Beispiel A2: {(R)-1-[(S)-2-Diphenylphosphino)ferrocenyl]}ethyl-di(3,5-bis-(trifluoromethyl)phenyl)phosphin (B).

Es wird analog Beispiel A1 verfahren und die Reaktionsbedingungen wie folgt abgewandelt:
0,85 g (1,92 mmol) N-{(R-1-[(S)-diphenylphosphino)ferrocenyl]}ethyl-dimethylamin, 1,0 g (2,18 mmol) Di(3,5-bis-(trifluoromethyl)phenyl)phosphin und 17 ml Essigsäure. Die Ausbeute beträgt 1,30 g **B** (Ausbeute: 80%) als orange kristalline Substanz.
³¹P-NMR (CDCl₃): -27,4 (d, J=30), 8,8 (d, J=30).

### Beispiel A3: {(R)-1-[(S)-2-Di-(4-trifluoromethylphenyl)-phosphino)ferrocenyl]}ethyl-di-(4-trifluoromethylphenyl)phosphin (C).

Es wird analog Beispiel A1 verfahren und die Reaktionsbedingungen wie folgt abgewandelt:
4,28 g (7,42 mmol) N-{(R)-1-[(S)-Di(4-trifluoromethylphenyl)phosphino)ferrocenyl]}ethyl-dimethylamin [hergestellt aus N-[(R)-1-Ferrocenyl]ethyl-dimethylamin und Di(4-trifluoromethylphenyl)phosphinchlorid analog Bull. Chem. Soc. Jpn., **53**, 1138 (1980)], 2,75 g (8,53 mmol) Di(4-trifluoromethylphenyl)phosphin und 55 ml Essigsäure. Die Ausbeute beträgt 5,89 g C (Ausbeute: 93%).
³¹P-NMR (CDCl₃): -25,7 (d, J=30), 8,4 (d, J=30)

### Beispiel A4: {(R)-1-[(S)-2-Di-(4-trifluoromethylphenyl)-phosphino)ferrocenyl]}ethyl-di-(3,5-dimethylphenyl)phosphin (D).

Es wird analog Beispiel A1 verfahren und die Reaktionsbedingungen wie folgt abgewandelt:
1,21 g (2,1 mmol) N-{(R)-1-[(S)-Di(4-trifluoromethylphenyl)phosphino)ferrocenyl]}ethyl-dimethylamin [hergestellt aus N-[(R)-1-Ferrocenyl]ethyl-dimethylamin und Di(4-trifluoromethylphenyl)phosphinchlorid analog Bull. Chem. Soc. Jpn., **53**, 1138 (1980)], 0,53 g (2,2 mmol) Di-(3,5-dimethylphenyl)phosphin und 15 ml Essigsäure. Die Ausbeute beträgt 460 mg als orange kristalline Substanz (**D**) (Ausbeute: 28%).
³¹P-NMR (CDCl₃): -24,7 (d, J=23), 8,7 (d, J=23).

### Beispiel A5: {(R)-1-[(S)-2-Di-(4-trifluoromethylphenyl)-phosphino)ferrocenyl]}ethyl-dicyclohexylphosphin (E).

Es wird analog Beispiel A1 verfahren und die Reaktionsbedingungen wie folgt abgewandelt:
1,73 g (3,0 mmol) N-{(R)-1-[(S)-Di(4-trifluoromethylphenyl)phosphino)ferrocenyl]}-ethyl-dimethylamin [hergestellt aus N-[(R)-1-Ferrocenyl]ethyl-dimethylamin und Di(4-trifluoromethylphenyl)phosphinchlorid analog Bull. Chem. Soc. Jpn., **53**, 1138 (1980)], 0,69 g (3,4 mmol) Dicyclohexylphosphin und 20 ml Essigsäure. Die Ausbeute beträgt 1,69 g als orange kristalline Substanz (**E**) (Ausbeute: 77%).
³¹P-NMR (CDCl₃): -25,5 (d, J=42), 15,65 (d, J=42).

### Beispiel A6: {(R)-1-[(S)-2-Di(3,5-bis-(trifluoromethyl)phenyl)phosphino)ferrocenyl]}ethyl-di(3,5-dimethylphenyl)phosphin (F)

Es wird analog Beispiel A1 verfahren und die Reaktionsbedingungen wie folgt abgewandelt.
1,07 g (1,5 mmol) N-{(R)-1-[(S)-Di(3,5-bis-(trifluoromethyl)phenyl)phosphino)ferrocenyl]}ethyl-dimethylamin [hergestellt analog Bull. Chem. Soc. Jpn., **53**, 1138 (1980)], 0,41 g (1,7 mmol) Di(3,5-dimethylphenyl)phosphin und 15 ml Essigsäure. Die Ausbeute beträgt 0,95 g **F** (69,3 % d. Th.) als orange kristalline Substanz.
³¹P-NMR (CDCl₃): -22,8 (d, J=35), 11,7 (d, J=35).

### Beispiel A7: {(R)-1-[(S)-2-Di(3,5-bis-(trifluoromethyl)phenyl)phosphino)ferrocenyl]}ethyl-diphenylphosphin (G)

Es wird analog Beispiel A1 verfahren und die Reaktionsbedingungen wie folgt abgewandelt.
1,0 g (1,4 mmol) N-{(R)-1-[(S)-Di(3,5-bis-(trifluoromethyl)phenyl)phosphino)ferrocenyl]}ethyl-dimethylamin [hergestellt analog Bull. Chem. Soc. Jpn., **53**, 1138 (1980)], 0,3 g (1,6 mmol) Diphenylphosphin und 14 ml Essigsäure. Die Ausbeute beträgt 1,1 g **G** (91,7 % d. Th.) als orange kristalline Substanz.
³¹P-NMR (CDCl₃): -22,9 (d, J=36), 10,7 (d, J=36).

### Beispiel A8: {(R)-1-[(S)-2-Di(3,5-bis-(trifluoromethyl)phenyl)phosphino)ferrocenyl]}ethyl-di(3-methoxyphenyl)phosphin (H)

Es wird analog Beispiel A1 verfahren und die Reaktionsbedingungen wie folgt abgewandelt.
1,0 g (1,4 mmol) N-{(R)-1-[(S)-Di(3,5-bis-(trifluoromethyl)phenyl)phosphino)ferrocenyl]}ethyl-dimethylamin [hergestellt analog Bull. Chem. Soc. Jpn., **53**, 1138 (1980)], 0,39 g (1,6 mmol) Di(3-methoxyphenyl)phosphin und 15 ml Essigsäure. Die Ausbeute beträgt 0,37 g **H** (30,5 % d. Th.) als oranges Harz, welches anschliessend aus Ether geschäumt wird.
³¹P-NMR (CDCl₃): -23,0 (d, J=33), 12,2 (d, J=33).

### Beispiel A9: {(R)-1-[(S)-2-Di(3,5-dimethylphenyl)phosphino)ferrocenyl]}ethyl-di(3,5-bis-(trifluoromethyl)phenyl)phosphin (I)

Es wird analog Beispiel A1 verfahren und die Reaktionsbedingungen wie folgt abgewandelt.
1,0 g (2,0 mmol) N-{(R)-1-[(S)-Di(3,5-dimethylphenyl)phosphino)ferrocenyl]}ethyldimethylamin [hergestellt analog Bull. Chem. Soc. Jpn., **53**, 1138 (1980)], 1,05 g (2,3 mmol) Di(3,5-bis-(trifluoromethyl)phenyl)phosphin und 15 ml Essigsäure. Die Ausbeute beträgt 310 mg **I** (17 % d. Th.) als orange kristalline Substanz.
³¹P-NMR (CDCl₃): -27,1 (d, J=30), 9,3 (d, J=30).

### Beispiel A10: {(R)-1-[(S)-2-Di(4-trifluoromethylphenyl)phosphino)ferrocenyl]}ethyl-diphenylphosphin (J)

Es wird analog Beispiel A1 verfahren und die Reaktionsbedingungen wie folgt abgewandelt.
1,04 g (1,8 mmol) N-{(R)-1-[(S)-Di(4-trifluoromethylphenyl)phosphino)ferrocenyl]}ethyl-dimethylamin [hergestellt analog Bull. Chem. Soc. Jpn., **53**, 1138 (1980)], 0,37 g (1,8 mmol) Diphenylphosphin und 15 ml Essigsäure. Die Ausbeute beträgt 1,03 g **J** (80 % d. Th.) als orange kristalline Substanz.
³¹P-NMR (CDCl₃): -25,0 (d, J=27), 8,4 (d, J=27).

### Beispiel A11: {(R)-1-[(S)-2-Di(4-trifluoromethylphenyl)phosphino)ferrocenyl]}ethyl-di(4-tert.-butyl-phenyl)phosphin (K)

Es wird analog Beispiel A1 verfahren und die Reaktionsbedingungen wie folgt abgewandelt.
1,15 g (1,9 mmol) N-{(R)-1-[(S)-Di(4-trifluoromethylphenyl)phosphino)ferrocenyl]}ethyl-dimethylamin [hergestellt analog Bull. Chem. Soc. Jpn., **53**, 1138 (1980)], 0,7 g (2,1 mmol) Di(4-tert.-butylphenyl)phosphin und 15 ml Essigsäure. Die Ausbeute beträgt 170 mg **K** (10 % d. Th.) als orange kristalline Substanz.
³¹P-NMR (CDCl₃): -24,5 (d, J=30), 6,6 (d, J=30).

### Anwendungsbeispiele

### Beispiel B1: Herstellung von N-(2'-Methyl-6'-ethyl-phen-1'-yl)-N-(1-methoxymethyl)ethylamin

Zu einer Lösung von 10,2 mg (0,015 mmol) [Ir(1,5-Cyclooctadien)Cl]₂ in 10 ml eines 3:1-Lösungsmittelgemisches von Tetrahydrofuran und Methylenchlorid wird 24,1 mg (0,033 mmol) **A** eingetragen und während 15 Minuten gerührt. Separat werden 5 ml (2,4 mmol) N-(2'-Methyl-6'-ethyl-phen-1'-yl)-N-(1-methoxymethyl)eth-1-ylidenamin in 10 ml Tetrahydrofuran/Methylenchlorid (3:1) gelöst. Die Imin- und die Katalysatorlösung werden nacheinander mit einer Stahlkapillare in einen unter Inertgas stehenden 50 ml-Stahlautoklaven transferiert. In drei Zyklen (20 bar, Normaldruck) wird das Inertgas durch Wasserstoff verdrängt. Anschliessend werden 25 bar Wasserstoff aufgepresst und der Autoklav auf 30°C erwärmt. Nach 6 Stunden wird die Reaktion abgebrochen. Der Umsatz beträgt 100%, die optische Ausbeute 56% (S).

### Beispiel B2: Herstellung von N-(2'-Methyl-6'-ethyl-phen-1'-yl)-N-(1-methoxymethyl)ethylamin

Eine Mischung von 10,2 mg (0,015 mmol) [Ir(1,5-Cyclooctadien)Cl]₂ und 28,5 mg (0,033 mmol) **B** wird mit 5 ml (24 mmol) N-(2'-Methyl-6'-ethyl-phen-1'-yl)-N-(1-methoxymethyl)eth-1-ylidenamin überschichtet und anschliessend mit einer Stahlkapillare in einen 50 ml-Stahlautoklav transferiert. Dann wird analog Beispiel B1 weiter verfahren.
Nach 24 Std. wird die Reaktion abgebrochen. Der Umsatz beträgt 87%, die Enantiomerenreinheit 62% (S).

Die in Tabelle 1 und 2 zusammengefassten Beispiele B3 - B17 werden analog dem Beispiel B1 durchgeführt.
Die folgenden Abkürzungen werden in Tabelle 1 und 2 verwendet:
Rh⁺=[Rh(Norbornadien)₂]BF₄, Rh⁰= [Rh(Norbornadien)Cl]₂,
Ir⁰=[Ir(1,5-Cyclooctadien)Cl]₂, S1: Methyl-Z-2-acetamidocinnamat, S2: Z-2-Acetamidozimtsäure, S3: Dimethylitaconat, S4: Methylacetylacetat, S5: Ethylpyruvat; S6: N-(2'-Methyl-6'ethyl-phen-1'-yl)-N-(1-methoxymethyl)eth-1-ylidenamin, M/T: MeOH/Toluol (1:1), *) Zusatz von 150 mg Tetrabutylammoniumiodid.

**Tabelle 1**

| Bsp. | Substrat(mmol) | Kat. | Ligand | S/C | Lsm. | bar H₂ |
|---|---|---|---|---|---|---|
| B3 | S6 (24,4) | Ir⁰ | C | 800 | - | 30 |
| B4 | S6 (24,4) | Ir⁰ | D | 800 | i-PrOH | 25 |
| B5 | S1 (3,4) | Rh⁺ | E | 100 | MeOH | 1 |
| B6 | S2 (3,4) | Rh⁺ | E | 100 | MeOH | 1 |
| B7 | S3 (3,14) | Rh⁺ | E | 200 | MeOH | 1 |
| B8 | S5 (4,4) | Rh⁰ | E | 200 | Toluol | 40 |
| B9 | S4 (4,3) | Rh⁰ | E | 200 | M/T | 20 |
| B10 | S1 (3,4) | Rh⁺ | G | 100 | MeOH | 50 |
| B11 | S2 (3,4) | Rh⁺ | G | 100 | MeOH | 50 |
| B12 | S1 (3,4) | Rh⁺ | H | 100 | MeOH | 50 |
| B13 | S4 (4,3) | Rh⁰ | B | 200 | M/T | 70 |
| B14 | S4 (4,3) | Rh⁰ | A | 200 | MeOH | 20 |
| B15 | S6 (24,4) | Ir^{0∗)} | F | 800 | iPrOH | 25 |
| B16 | S6 (24,4) | Ir^{0∗)} | B | 800 | - | 30 |
| B17 | S6 (24,4) | Ir^{0∗)} | G | 800 | i-PrOH | 25 |

**Tabelle 2**

| Bsp. | Substrat(mmol) | T[°C] | Zeit Std | Umsatz % | e.e.(%) | (Konfig.) |
|---|---|---|---|---|---|---|
| B3 | S6 (24,4) | 25 | 24 | 82 | 67,4 | (S) |
| B4 | S6 (24,4) | 25 | 20 | 99 | 79,8 | (S) |
| B5 | S1 (3,4) | 25 | 1 | 100 | 98 | (R) |
| B6 | S2 (3,4) | 25 | 0,5 | 100 | 96,3 | (R) |
| B7 | S3 (3,14) | 25 | 0,5 | 100 | >99,5 | (S) |
| B8 | S5 (4,4) | 10 | 41 | 91,5 | 56 | (S) |
| B9 | S4 (4,3) | 25 | 44 | 98 | 77,3 | (S) |
| B10 | S1 (3,4) | 40 | 65 | 96 | 83,5 | (R) |
| B11 | S2 (3,4) | 25 | 10 | 100 | 73,6 | (R) |
| B12 | S1 (3,4) | 40 | 21 | 100 | 73,6 | (R) |
| B13 | S4 (4,3) | 25 | 93 | 98 | 52,3 | (S) |
| B14 | S4 (4,3) | 25 | 85 | 50 | 78 | (S) |
| B15 | S6 (24,4) | 25 | 18 | 100 | 75,8 | (S) |
| B16 | S6 (24,4) | 25 | 24 | 87 | 61,6 | (S) |
| B17 | S6 (24,4) | 25 | 18 | 100 | 78,2 | (S) |

## Patentansprüche

1. Verbindungen der Formel I, 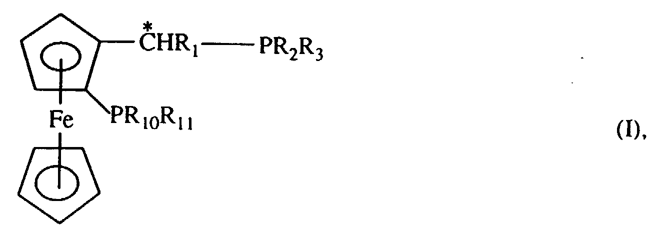 worin R₁ C₁-C₈-Alkyl, Phenyl oder mit 1 bis 3 C₁-C₄-Alkyl oder C₁-C₄-Alkoxy substituiertes Phenyl bedeutet; R₂ einen Rest der Formel II 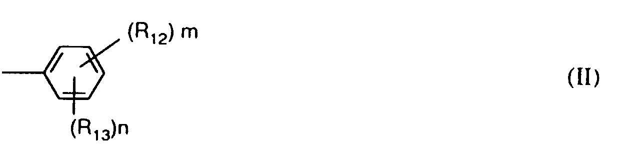 darstellt, worin R₁₂ ein teilweise oder vollständig mit Fluor substituiertes C₁-C₅-Alkyl bedeutet;
R₁₃ C₁-C₄-Alkyl, C₁-C₄-Alkoxy, -SiR₄R₅R₆, Halogen, -SO₃M, -CO₂M, -PO₃M, -NR₇R₈ oder -[^{⊕}NR₇R₈R₉]X^{⊖} ist;
m eine Zahl von 1 bis 3 ist, n für 0 oder eine Zahl von 1 bis 4 steht und die Summe von m+n 1 bis 5 ist;
R₃, R₁₀, R₁₁ unabhängig voneinander die gleiche Bedeutung wie R₂ haben oder unabhängig voneinander C₁-C₁₂-Alkyl, C₅-C₁₂-Cycloalkyl, Phenyl, mit C₁-C₄-Alkyl oder C₁-C₄-Alkoxy substituiertes C₅-C₁₂-Cycloalkyl oder mit ein bis drei C₁-C₄-Alkyl, C₁-C₄-Alkoxy, -SiR₄R₅R₆, Halogen, -SO₃M, -CO₂M, -PO₃M, -NR₇R₈ oder -[^{⊕}NR₇R₈R₉]X^{⊖} substituiertes Phenyl darstellen;
R₄, R₅ und R₆ unabhängig voneinander für C₁-C₁₂-Alkyl oder Phenyl stehen;
R₇ und R₈ H, C₁-C₁₂-Alkyl, Phenyl oder
R₇ und R₈ zusammen Tetramethylen, Pentamethylen oder 3-Oxa-1,5-Pentylen darstellen;
R₉ H oder C₁-C₄-Alkyl bedeutet,
M für H oder ein Alkalimetall steht, X^{⊖} das Anion einer einbasischen Säure ist, und * ein stereogenes C-Atom kennzeichnet, in Form ihrer Racemate und Diastereomeren oder Gemischen von Diastereomeren, mit Ausnahme von {(R)-1-[(S)-2-Diphenylphosphino)ferrocenyl]} ethyl-di-(4-trifluoromethylphenyl)phosphin.

2. Verbindungen der Formel I gemäss Anspruch 1, dadurch gekennzeichnet, dass R₁ als Alkyl linear ist.

3. Verbindungen der Formel I gemäss Anspruch 2, dadurch gekennzeichnet, dass R₁ C₁-C₄-Alkyl darstellt.

4. Verbindungen der Formel I gemäss Anspruch 3, dadurch gekennzeichnet, dass R₁ für Methyl oder Ethyl steht.

5. Verbindungen der Formel I gemäss Anspruch 1, dadurch gekennzeichnet, dass R₁ Phenyl oder mit 1 oder 2 C₁-C₄-Alkyl oder C₁-C₄-Alkoxy substituiertes Phenyl darstellt.

6. Verbindungen der Formel I gemäss Anspruch 1, dadurch gekennzeichnet, dass R₃, R₁₀ und R₁₁ C₁-C₈-Alkyl darstellen.

7. Verbindungen der Formel I gemäss Anspruch 1, dadurch gekennzeichnet, dass R₃, R₁₀ und R₁₁ als Cycloalkyl 5 bis 8 Ring-C-Atome enthalten.

8. Verbindungen der Formel I gemäss Anspruch 7, dadurch gekennzeichnet, dass R₃, R₁₀ und R₁₁ als Cycloalkyl Cyclopentyl oder Cyclohexyl darstellt.

9. Verbindungen der Formel I gemäss Anspruch 1, dadurch gekennzeichnet, dass R₃, R₁₀ und R₁₁ Phenyl, 2-Methyl-, 3-Methyl-, 4-Methyl-, 2- oder 4-Ethyl-, 2- oder 4-i-Propyl-, 2- oder 4-t-Butyl-, 2-Methoxy-, 3-Methoxy-, 4-Methoxy-, 2- oder 4-Ethoxy-, 4-Trimethylsilyl-, 2- oder 4-Fluor-, 2,4-Difluor-, 2- oder 4-Chlor-, 2,4-Dichlor-, 2,4-Dimethyl-, 3,5-Dimethyl-, 2-Methoxy-4-methyl-, 3,5-Dimethyl-4-methoxy-, 3,5-Dimethyl-4-(dimethylamino)-, 2- oder 4-Amino-, 2- oder 4-Methylamino-, 2- oder 4-(Dimethylamino)-, 2- oder 4-SO₃H-, 2- oder 4-SO₃Na-, 2- oder 4-[^{⊕}NH₃Cl^{⊖}]-, 2,4,6-Trimethylphen-1-yl oder 3,4,5-Trimethylphen-1-yl darstellen.

10. Verbindungen der Formel I gemäss Anspruch 1, dadurch gekennzeichnet, dass R₁₂ C₁-C₃ Perfluoralkyl ist.

11. Verbindungen der Formel I gemäss Anspruch 1, dadurch gekennzeichnet, dass R₁₃ C₁-C₄-Alkyl oder C₁-C₄- Alkoxy ist.

12. Verbindungen der Formel I gemäss Anspruch 1, dadurch gekennzeichnet, dass R₁₂ 4-Trifluormethyl oder 3, 5- Di-(trifluormethyl) bedeutet.

13. Verbindungen der Formel I gemäss Anspruch 1, dadurch gekennzeichnet, dass R₃, R₁₀ und R₁₁ gleiche Reste darstellen und Phenyl, Cyclohexyl, t-Butyl, 2- oder 4-Methylphen-1-yl, 2- oder 4-Methoxyphen-1-yl, 2- oder 4-(Dimethylamino)phen-1-yl, 3,5-Dimethyl-4-(dimethylamino)phen-1-yl oder 3,5-Dimethyl-4-methoxyphen-1-yl bedeuten.

14. Verbindungen der Formel I gemäss Anspruch 1, dadurch gekennzeichnet, dass R₂ und R₃ gleich sind und einen Rest der Formel II darstellen und R₁₀ und R₁₁ gleiche Reste darstellen und Phenyl Cyclohexyl, t-Butyl, 2- oder 4-Methylphen-1-yl, 2- oder 4-Methoxyphen-1-yl, 2- oder 4-(Dimethylamino)phen-1-yl, 3,5-Dimethyl-4-(dimethylamino)phen-1-yl oder 3,5-Dimethyl-4-methoxyphen-1-yl bedeuten.

15. Verbindungen der Formel I gemäss Anspruch 1, dadurch gekennzeichnet, dass R₂ und R₃ gleich sind und einen Rest der Formel II darstellen, R₁₀ Phenyl und R₁₁ Cyclohexyl, 2- oder 4-Methylphen-1-yl, 2- oder 4-Methoxyphen-1-yl, 4-(Dimethylamino)phen-1-yl, 3,5-Dimethyl-4-(dimethylamino)phen-1-yl, 3,5-Dimethyl-4-methoxyphen-1-yl oder 4-t-Butyl-phen-1-yl bedeuten.

16. Verbindungen der Formel I gemäss Anspruch 1, dadurch gekennzeichnet, dass R₁ Methyl ist, R₂ und R₃ gleich sind und einen Rest der Formel II darstellen, R₁₀ und R₁₁ gleiche Reste darstellen und Phenyl oder Cyclohexyl bedeuten.

17. Verbindungen der Formel I gemäss Anspruch 1, dadurch gekennzeichnet, dass R₂, R₃, R₁₀ und R₁₁ gleich sind und einen Rest der Formel II bedeuten.

18. Verbindungen der Formel I gemäss Anspruch 1, dadurch gekennzeichnet, dass R₁ Methyl ist, R₂, R₃, R₁₀ und R₁₁ gleich sind und einen Rest der Formel II darstellen, R₁₂ 4-Trifluormethyl oder 3,5-Di(trifluormethyl) bedeutet und n 0 ist.

19. Verbindungen der Formel I gemäss Anspruch 1, dadurch gekennzeichnet, dass R₁ Methyl ist, R₂ und R₃ gleich sind und einen Rest der Formel II darstellen, R₁₀ und R₁₁ Phenyl sind, R₁₂ 4-Trifluormethyl oder 3,5-Di(trifluormethyl) bedeutet und n 0 ist.

20. Verbindungen der Formel I gemäss Anspruch 1, dadurch gekennzeichnet, dass es sich um
{(R)-1-[(S)-2-(Di-(4-trifluoromethylphenyl)-phosphino)ferrocenyl]}ethyl-di(3,5-dimethylphenyl)phosphin,
{(R)-1-[(S)-2-(Di-(4-trifluoromethylphenyl)-phosphino)ferrocenyl]}ethyl-dicyclo-hexylphosphin,
{(R)-1-[(S)-2-(Di-(3,5-bis-(trifluoromethyl)phenyl)phosphino)ferrocenyl]}ethyl-di(3,5-dimethylphenyl)phosphin,
{(R)-1-[(S)-2-(Di-(3,5-bis-(trifluoromethyl)phenyl)phosphino)ferrocenyl]}ethyl-diphenyl-phosphin oder
{(R)-1-[(S)-2-(Di-(4-trifluoromethylphenyl)phosphino)ferrocenyl]}ethyl-diphenylphosphin handelt.

21. Verfahren zur Herstellung von Verbindungen der Formel I gemäss Anspruch 1, dadurch gekennzeichnet, dass man
entweder eine Verbindung der Formel III 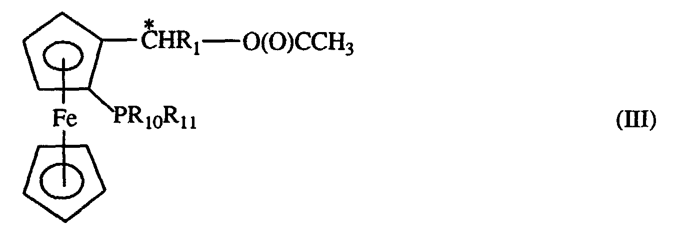 in Gegenwart eines inerten Lösungsmittels bei Raumtemperatur oder erhöhter Temperatur mit einem Phosphin der Formel IV
HPR₂R₃ (IV)
umsetzt; oder eine Verbindung der Formel VII 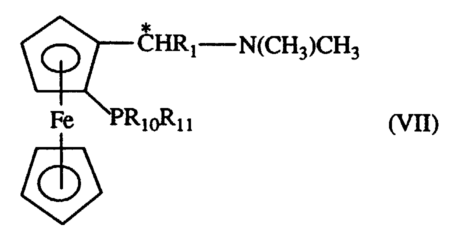 worin R₁, R₁₀ und R₁₁ die unter Formel I angegebenen Bedeutungen haben, in Gegenwart eines inerten Lösungsmittels bei Raumtemperatur oder erhöhter Temperatur mit einem Phosphin der Formel IV
HPR₂R₃ (IV)
umsetzt, wobei R₁, R₂, R₃, R₁₀ und R₁₁ die in Anspruch 1 angegebenen Bedeutungen haben.

22. Komplexe der Formeln V und VI,
[X₁M₁YZ] (V),
[X₁M₁Y]^{⊕}A₁^{⊖} (VI),
worin X₁ für zwei C₂-C₁₂-Olefine oder ein C₅-C₁₂-Dien steht, Z für Cl, Br oder I steht, A₁^{^{⊖}} das Anion einer Sauerstoffsäure oder Komplexsäure bedeutet, M₁ für Rh oder Ir steht und Y eine Verbindung der Formel I gemäss Anspruch 1 darstellt.

23. Komplexe gemäss Anspruch 22, dadurch gekennzeichnet, dass es sich um solche der Formel VI handelt.

24. Komplexe gemäss Anspruch 22, dadurch gekennzeichnet, dass X₁ zwei Ethylen, 1,5-Hexadien, 1,5-Cyclooctadien oder Norbornadien darstellen.

25. Komplexe gemäss Anspruch 22, dadurch gekennzeichnet, dass Z für Cl oder Br steht.

26. Komplexe gemäss Anspruch 22, dadurch gekennzeichnet, dass Z für ClO₄^{^{⊖}}, FSO₃^{^{⊖}}, CH₃SO₃^{^{⊖}} , CF₃SO₃^{^{⊖}} , BF₄^{^{⊖}}, PF₆^{^{⊖}}, SbCl₆^{^{⊖}}, AsF₆^{^{⊖}} und SbF₆^{^{⊖}} steht.

27. Komplexe gemäss Anspruch 22, dadurch gekennzeichnet, dass R₁ als Alkyl linear ist.

28. Komplexe gemäss Anspruch 27, dadurch gekennzeichnet, dass R₁ C₁-C₄-Alkyl darstellt.

29. Komplexe gemäss Anspruch 28, dadurch gekennzeichnet, dass R₁ für Methyl oder Ethyl steht.

30. Komplexe gemäss Anspruch 22, dadurch gekennzeichnet, dass R₁ Phenyl oder mit 1 oder 2 C₁-C₄-Alkyl oder C₁-C₄-Alkoxy substituiertes Phenyl darstellt.

31. Komplexe gemäss Anspruch 22, dadurch gekennzeichnet, dass R₃, R₁₀ und R₁₁ C₁-C₈-Alkyl darstellen.

32. Komplexe gemäss Anspruch 22, dadurch gekennzeichnet, dass R₃, R₁₀ und R₁₁ als Cycloalkyl 5 bis 8 Ring-C-Atome enthält.

33. Komplexe gemäss Anspruch 32, dadurch gekennzeichnet, dass R₃, R₁₀ und R₁₁ als Cycloalkyl Cyclopentyl oder Cyclohexyl darstellt.

34. Komplexe gemäss Anspruch 22, dadurch gekennzeichnet, dass R₃, R₁₀ und R₁₁ Phenyl, 2-Methyl-, 3-Methyl-, 4-Methyl-, 2- oder 4-Ethyl-, 2- oder 4-i-Propyl-, 2- oder 4-t-Butyl-, 2-Methoxy-, 3-Methoxy-, 4-Methoxy-, 2- oder 4-Ethoxy-, 4-Trimethylsilyl-, 2- oder 4-Fluor-, 2,4-Difluor-, 2- oder 4-Chlor-, 2,4-Dichlor-, 2,4-Dimethyl-, 3,5-Dimethyl-, 2-Methoxy-4-methyl-, 3,5-Dimethyl-4-methoxy-, 3,5-Dimethyl-4-(dimethylamino)-, 2- oder 4-Amino-, 2- oder 4-Methylamino-, 2- oder 4-(Dimethylamnio)-, 2- oder 4-SO₃H-, 2- oder 4-SO₃Na-, 2- oder 4-[^{⊕} NH₃Cl^{^{⊖}}]-, 2,4,6-Trimethylphen-1-yl oder 3,4,5-Trimethylphen-1-yl darstellen.

35. Komplexe gemäss Anspruch 22, dadurch gekennzeichnet, dass R₁₂ C₁-C₃ Perfluoralkyl ist.

36. Komplexe gemäss Anspruch 22, dadurch gekennzeichnet, dass R₁₃ C₁-C₄-Alkyl, C₁-C₄- Alkoxy ist.

37. Komplexe gemäss Anspruch 22, dadurch gekennzeichnet, dass R₁₂ 4-Trifluormethyl oder 3, 5- Di-(trifluormethyl) bedeutet.

38. Komplexe gemäss Anspruch 22, dadurch gekennzeichnet, dass R₃, R₁₀ und R₁₁ gleiche Reste darstellen und Phenyl, Cyclohexyl, t-Butyl, 2- oder 4-Methylphen-1-yl, 2- oder 4-Methoxyphen-1-yl, 2- oder 4-(Dimethylamino)phen-1-yl, 3,5-Dimethyl-4-(dimethylamino)phen-1-yl oder 3,5-Dimethyl-4-methoxyphen-1-yl bedeuten.

39. Komplexe gemäss Anspruch 22, dadurch gekennzeichnet, dass R₂ und R₃ gleich sind und einen Rest der Formel II darstellen und R₁₀ und R₁₁ gleiche Reste darstellen und Phenyl, Cyclohexyl, t-Butyl, 2- oder 4-Methylphen-1-yl, 2- oder 4-Methoxyphen-1-yl, 2- oder 4-(Dimethylamino)phen-1-yl, 3,5-Dimethyl-4-(dimethylamino)phen-1-yl oder 3,5-Dimethyl-4-methoxyphen-1-yl bedeuten.

40. Komplexe gemäss Anspruch 22, dadurch gekennzeichnet, dass R₂ und R₃ gleich sind und einen Rest der Formel II bedeuten, R₁₀ Phenyl und R₁₁ Cyclohexyl, 2- oder 4-Methylphen-1-yl, 2- oder 4-Methoxyphen-1-yl, 4-(Dimethylamino)phen-1-yl, 3,5-Dimethyl-4-(dimethylamino)phen-1-yl, 3,5-Dimethyl-4-methoxyphen-1-yl oder 4-t-Butyl-phen-1-yl bedeuten.

41. Komplexe gemäss Anspruch 22, dadurch gekennzeichnet, dass R₁ Methyl ist, R₂ und R₃ gleich sind und einen Rest der Formel II darstellen, R₁₀ und R₁₁ gleiche Reste darstellen und Phenyl oder Cyclohexyl bedeuten.

42. Komplexe gemäss Anspruch 22, dadurch gekennzeichnet, dass R₂, R₃, R₁₀ und R₁₁ gleich sind und einen Rest der Formel II bedeuten.

43. Komplexe gemäss Anspruch 22, dadurch gekennzeichnet, dass, R₁ Methyl ist, R₂, R₃, R₁₀ und R₁₁ gleich sind und einen Rest der Formel II darstellen, R₁₂ 4-Trifluormethyl oder 3,5-Di(trifluormethyl) bedeutet und n 0 ist.

44. Komplexe gemäss Anspruch 22, dadurch gekennzeichnet, dass R₁ Methyl ist, R₂ und R₃ gleich sind und einen Rest der Formel II darstellen, R₁₀ und R₁₁ Phenyl sind, R₁₂ 4-Trifluormethyl oder 3,5-Di(trifluormethyl) bedeutet und n 0 ist.

45. Komplexe gemäss Anspruch 22, dadurch gekennzeichnet, dass Y
{(R)-1-[(S)-2-(Di-(4-trifluoromethylphenyl)-phosphino)ferrocenyl]}ethyl-di(3,5-dimethylphenyl)phosphin,
{(R)-1-[(S)-2-(Di-(4-trifluoromethylphenyl)-phosphino)ferrocenyl]}ethyl-dicyclohexylphosphin,
{(R)-1-[(S)-2-(Di-(3,5-bis-(trifluoromethyl)phenyl)phosphino)ferrocenyl]}ethyl-di(3,5-dimethylphenyl)phosphin,
{(R)-1-[(S)-2-(Di-(3,5-bis-(trifluoromethyl)phenyl)phosphino)ferrocenyl]}ethyl-diphenylphosphin oder
{(R)-1-[(S)-2-(Di-(4-trifluoromethylphenylphosphino)ferrocenyl]}ethyl-diphenylphosphin ist.

46. Verwendung der Komplexe der Formeln V und VI gemäss Anspruch 22 als homogene Katalysatoren zur asymmetrischen Hydrierung von prochiralen Verbindungen mit Kohlenstoff- oder Kohlenstoff-/Heteroatomdoppelbindungen.

47. Verwendung gemäss Anspruch 46 zur Hydrierung von unsymmetrischen Kohlenstoffdoppelbindungen, Ketiminen und Ketonen.

48. Verwendung gemäss Anspruch 46 zur Hydrierung von unsymmetrischen Kohlenstoffdoppelbindungen mit den Rhodium-Komplexen der Formeln V und VI.

49. Verfahren zur asymmetrischen Hydrierung von Verbindungen mit Kohlenstoff- oder Kohlenstoff-/Heteroatomdoppelbindungen unter homogenen Reaktionsbedingungen, dadurch gekennzeichnet, dass man die Verbindungen bei einer Temperatur von -20 bis 80 °C und einem Wasserstoffdruck von 10⁴ bis 10⁷ Pa in Gegenwart katalytischer Mengen eines Komplexes der Formeln V oder VI gemäss Anspruch 22 hydriert.

50. Verfahren gemäss Anspruch 49, dadurch gekennzeichnet, dass der Wasserstoffdruck 10⁵ bis 6·10⁶ Pa beträgt.

51. Verfahren gemäss Anspruch 49, dadurch gekennzeichnet, dass die Temperatur -10 bis 50 °C beträgt.

52. Verfahren gemäss Anspruch 49, dadurch gekennzeichnet, dass die Menge des Katalysators so gewählt wird, dass das Molverhältnis der zu hydrierenden Verbindung (Substrat) zu dem Komplex der Formeln V oder VI 100000 bis 20 beträgt.

53. Verfahren gemäss Anspruch 49, dadurch gekennzeichnet, dass die Reaktion in Gegenwart eines Lösungsmittels durchgeführt wird.

54. Verfahren gemäss Anspruch 49, dadurch gekennzeichnet, dass das Lösungsmittel Methanol oder eine Mischung von Methanol mit Benzol oder Toluol ist.

## Claims

1. Compounds of formula I, 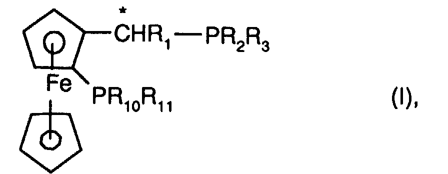 wherein R₁ is C₁-C₈-alkyl, phenyl or phenyl which is substituted by 1 to 3 C₁-C₄-alkyl or C₁-C₄-alkoxy groups; R₂ is a radical of formula II 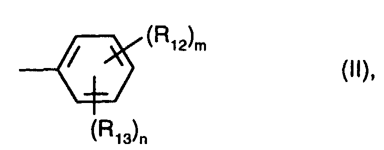 wherein R₁₂ is C₁-C₅-alkyl which is partially or completely fluorinated;
R₁₃ is C₁-C₄-alkyl, C₁-C₄-alkoxy, -SiR₄R₅R₆, halogen, -SO₃M, -CO₂M, -PO₃M, -NR₇R₈ or -[^{⊕}NR₇R₈R₉]X^{^{⊖}};
m is an integer from 1 to 3, n is 0 or an integer from 1 to 4, and the sum of m+n is 1 to 5;
R₃, R₁₀, R₁₁, independently of one another, have the same significance as R₂ or, independently of one another, are each C₁-C₁₂-alkyl, C₅-C₁₂-cydoalkyl, phenyl, C₁-C₄-alkyl- or C₁-C₄-alkoxy-substituted C₅-C₁₂-cycloalkyl, or phenyl which is mono- to tri-substituted by C₁-C₄-alkyl, C₁-C₄-alkoxy, -SiR₄R₅R₆, halogen, -SO₃M, -CO₂M, -PO₃M, -NR₇R₈ or -[^{⊕}NR₇R₈R₉]X^{^{⊖}} ;
R₄, R₅ and R₆, independently of one another, are C₁-C₁₂-alkyl or phenyl;
R₇ and R₈ are H, C₁-C₁₂-alkyl or phenyl or
R₇ and R₈ taken together are tetramethylene, pentamethylene or 3-oxa-1,5-pentylene;
R₉ is H or C₁-C₄-alkyl,
M is H or an alkali metal, X^{^{⊖}} is the anion of a monobasic acid, and * is a stereogenic carbon atom, in the form of their racemates and diastereoisomers or mixtures of diastereoisomers, with the exception of {(R)-1-[(S)-2-(diphenylphosphino)ferrocenyl]}ethyl bis(4-trifluoromethylphenyl)phosphine.

2. Compounds of formula I according to claim 1, wherein R₁ as alkyl is linear.

3. Compounds of formula I according to claim 2, wherein R₁ is C₁-C₄-alkyl.

4. Compounds of formula I according to claim 3, wherein R₁ is methyl or ethyl.

5. Compounds of formula I according to claim 1, wherein R₁ is phenyl or phenyl which is substituted by one or two C₁-C₄-alkyl or C₁-C₄-alkoxy groups.

6. Compounds of formula I according to claim 1, wherein R₃, R₁₀ and R₁₁ are C₁-C₈-alkyl.

7. Compounds of formula I according to claim 1, wherein R₃, R₁₀ and R₁₁ as cycloalkyl contain 5 to 8 ring carbon atoms.

8. Compounds of formula I according to claim 7, wherein R₃, R₁₀ and R₁₁ as cycloalkyl are cyclopentyl or cyclohexyl.

9. Compounds of formula I according to claim 1, wherein R₃, R₁₀ and R₁₁ are phenyl, 2-methyl-, 3-methyl-, 4-methyl-, 2- or 4-ethyl-, 2- or 4-isopropyl-, 2- or 4-tert.-butyl-, 2-methoxy-, 3-methoxy-, 4-methoxy-, 2- or 4-ethoxy-, 4-trimethylsilyl-, 2- or 4-fluoro-, 2,4-difluoro-, 2- or 4-chloro-, 2,4-dichloro-, 2,4-dimethyl-, 3,5-dimethyl-, 2-methoxy-4-methyl-, 3,5-dimethyl-4-methoxy-, 3,5-dimethyl-4-(dimethylamino)-, 2- or 4-amino-, 2- or 4-methylamino-, 2- or 4-(dimethylamino)-, 2- or 4-SO₃H-, 2- or 4-SO₃Na-, 2- or 4-[^{⊕}NH₃Cl^{^{⊖}}]-, 2,4,6-trimethyl-phen-1 -yl or 3,4,5-trimethyl-phen-1-yl.

10. Compounds of formula I according to claim 1, wherein R₁₂ is C₁-C₃-perfluoroalkyl.

11. Compounds of formula I according to claim 1, wherein R₁₃ is C₁-C₄-alkyl or C₁-C₄-alkoxy.

12. Compounds of formula I according to claim 1, wherein R₁₂ is 4-trifluoromethyl or 3,5-bis(trifluoromethyl).

13. Compounds of formula I according to claim 1, wherein R₃, R₁₀ and R₁₁ are identical substituents and are phenyl, cyclohexyl, tert.-butyl, 2- or 4-methylphen-1-yl, 2- or 4-methoxyphen-1-yl, 2 or 4-(dimethylamino)phen-1-yl, 3,5-dimethyl-4-(dimethylamino)phen-1-yl or 3,5-dimethyl-4-methoxyphen-1-yl.

14. Compounds of formula I according to claim 1, wherein R₂ and R₃ are identical substituents and are a radical of formula II, and R₁₀ and R₁₁ are identical substituents and are phenyl, cyclohexyl, tert.-butyl, 2- or 4-methylphen-1-yl, 2- or 4-methoxyphen-1-yl, 2 or 4-(dimethylamino)phen-1-yl, 3,5-dimethyl-4-(dimethylamino)phen-1-yl or 3,5-dimethyl-4-methoxyphen-1 -yl.

15. Compounds of formula I according to claim 1, wherein R₂ and R₃ are identical substituents and are a radical of formula II, R₁₀ is phenyl and R₁₁ is cyclohexyl, 2- or 4-methylphen-1-yl, 2- or 4-methoxyphen-1-yl, 4-(dimethylamino)phen-1-yl, 3,5-dimethyl-4-(dimethylamino)phen-1 -yl, 3,5-dimethyl-4-methoxyphen-1-yl or 4-tert.-butylphen-1-yl.

16. Compounds of formula I according to claim 1, wherein R₁ is methyl, R₂ and R₃ are identical and are a radical of formula II, R₁₀ and R₁₁ are identical substituents and are phenyl or cyclohexyl.

17. Compounds of formula I according to claim 1, wherein R₂, R₃, R₁₀ and R₁₁ are identical and are a radical of formula II.

18. Compounds of formula I according to claim 1, wherein R1 is methyl, R₂, R₃, R₁₀ and R₁₁ are identical and are a radical of formula II, R₁₂ is 4-trifluoromethyl or 3,5-bis(trifluoromethyl) and n is 0.

19. Compounds of formula I according to claim 1, wherein R₁ is methyl, R₂ and R₃ are identical and are a radical of formula II, R₁₀ and R₁₁ are phenyl, R₁₂ is 4-trifluoromethyl or 3,5-bis(trifluoromethyl) and n is 0.

20. Compounds of formula I according to claim 1, wherein they are selected from
{(R)-1-[(S)-2-(bis(4-trifluoromethylphenyl)-phosphino)ferrocenyl]}ethyl bis(3,5-dimethylphenyl)phosphine,
{(R)-1-[(S)-2-(bis(4-trifluoromethylphenyl)-phosphino)ferrocenyl]}ethyl-dicyclohexylphosphine,
{(R)-1-[(S)-2-(bis(3,5-bis-(trifluoromethyl)phenyl)phosphino)ferrocenyl]}ethyl bis(3,5-dimethylphenyl)phosphine,
{(R)-1-[(S)-2-(bis(3,5-bis-(trifluoromethyl)phenyl)phosphino)ferrocenyl]}ethyl-diphenylphosphine or
{(R)-1-[(S)-2-(bis(4-trifluoromethylphenyl)phosphino)ferrocenyl]}ethyl-diphenylphosphine.

21. Process for the preparation of compounds of formula I according to claim 1, which comprises
either reacting a compound of formula III 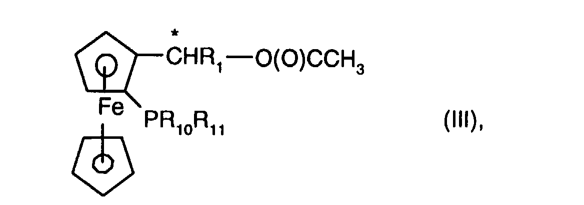 in the presence of an inert solvent, at room temperature or elevated temperature, with a phosphine of formula IV
HPR₂R₃ (IV);
or reacting a compound of formula VII 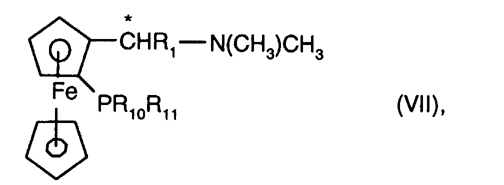 wherein R₁, R₁₀ and R₁₁ are as defined for formula I, in the presence of an inert solvent, at room temperature or elevated temperature, with a phosphine of formula IV
HPR₂R₃ (IV),
wherein R₁, R₂, R₃, R₁₀ and R₁₁ are as defined in claim 1.

22. Complexes of formulae V and VI,
[X₁M₁YZ] (V)
[X₁M₁Y]^{⊕}A₁^{⊖} (VI),
wherein X₁ is two C₂-C₁₂-olefins or one C₅-C₁₂-diene, Z is Cl, Br or I, A₁^{⊖} is the anion of an oxyacid or of a complex acid, M₁ is Rh or Ir, and Y is a compound of formula I as claimed in claim 1.

23. Complexes according to claim 22, wherein they are a complex of formula VI.

24. Complexes according to claim 22, wherein X₁ is two ethylene, 1,5-hexadiene, 1,5-cyclooctadiene or norbomadiene.

25. Complexes according to claim 22, wherein Z is Cl or Br.

26. Complexes according to claim 22, wherein Z is ClO₄^{⊖}, FSO₃^{⊖}, CH₃SO₃^{⊖}, CF₃SO₃^{⊖}, BF₄^{⊖}, PF₆^{⊖}, SbCl₆^{⊖}, AsF₆^{⊖} and SbF₆^{⊖}.

27. Complexes according to claim 22, wherein R₁ as alkyl is linear.

28. Complexes according to claim 27, wherein R₁ is C₁-C₄-alkyl.

29. Complexes according to claim 28, wherein R₁ is methyl or ethyl.

30. Complexes according to claim 22, wherein R₁ is phenyl or phenyl which is substituted by 1 or 2 C₁-C₄-alkyl or C₁-C₄-alkoxy groups.

31. Complexes according to claim 22, wherein R₃, R₁₀ and R₁₁ are C₁-C₈-alkyl.

32. Complexes according to claim 22, wherein R₃, R₁₀ and R₁₁ as cycloalkyl contain 5 to 8 ring carbon atoms.

33. Complexes according to claim 32, wherein R₃, R₁₀ and R₁₁ as cycloalkyl are cyclopentyl or cyclohexyl.

34. Complexes according to claim 22, wherein R₃, R₁₀ and R₁₁ are phenyl, 2-methyl-, 3-methyl-, 4-methyl-, 2- or 4-ethyl-, 2- or 4-isopropyl-, 2- or 4-tert.-butyl-, 2-methoxy-, 3-methoxy-, 4-methoxy-, 2- or 4-ethoxy-, 4-trimethylsilyl-, 2- or 4-fluoro-, 2,4-difluoro-, 2- or 4-chloro-, 2,4-dichloro-, 2,4-dimethyl-, 3,5-dimethyl-, 2-methoxy-4-methyl-, 3,5-dimethyl-4-methoxy-, 3,5-dimethyl-4-(dimethylamino)-, 2- or 4-amino-, 2- or 4-methylamino-, 2- or 4-(dimethylamino)-, 2-or 4-SO₃H-, 2- or 4-SO₃Na-, 2- or 4-[^{⊕}NH₃Cl^{⊖}]-, 2,4,6-trimethyl-phen-1-yl or 3,4,5-trimethylphen-1-yl.

35. Complexes according to claim 22, wherein R₁₂ is C₁-C₃-perfluoroalkyl.

36. Complexes according to claim 22, wherein R₁₃ is C₁-C₄-alkyl or C₁-C₄-alkoxy.

37. Complexes according to claim 22, wherein R₁₂ is 4-trifluoromethyl or 3,5-bis(trifluoromethyl).

38. Complexes according to claim 22, wherein R₃, R₁₀ and R₁₁ are identical substituents and are phenyl, cyclohexyl, tert.-butyl, 2- or 4-methylphen-1-yl, 2- or 4-methoxyphen-1-yl, 2 or 4-(dimethylamino)phen-1-yl, 3,5-dimethyl-4-(dimethylamino)phen-1-yl or 3,5-dimethyl-4-methoxyphen-1-yl.

39. Complexes according to claim 22, wherein R₂ and R₃ are identical substituents and are a radical of formula II, and R₁₀ and R₁₁ are identical substituents and are phenyl, cyclohexyl, tert.-butyl, 2- or 4-methylphen-1-yl, 2- or 4-methoxyphen-1-yl, 2 or 4-(dimethylamino)phen-1-yl, 3,5-dimethyl-4-(dimethylamino)phen-1-yl or 3,5-dimethyl-4-methoxyphen-1-yl.

40. Complexes according to claim 22, wherein R₂ and R₃ are identical substituents and are a radical of formula II, R₁₀ is phenyl and R₁₁ is cyclohexyl, 2- or 4-methylphen-1-yl, 2- or 4-methoxyphen-1-yl, 4-(dimethylamino)phen-1-yl, 3,5-dimethyl-4-(dimethylamino)phen-1-yl, 3,5-dimethyl-4-methoxyphen-1-yl or 4-tert.-butylphen-1-yl.

41. Complexes according to claim 22, wherein R₁ is methyl, R₂ and R₃ are identical and are a radical of formula II, R₁₀ and R₁₁ are identical substituents and are phenyl or cyclohexyl.

42. Complexes according to claim 22, wherein R₂, R₃, R₁₀ and R₁₁ are identical and are a radical of formula II.

43. Complexes according to claim 22, wherein R₁ is methyl, R₂, R₃, R₁₀ and R₁₁ are identical and are a radical of formula II, R₁₂ is 4-trifluoromethyl or 3,5-bis(trifluoromethyl) and n is 0.

44. Complexes according to claim 22, wherein R₁ is methyl, R₂ and R₃ are identical and are a radical of formula II, R₁₀ and R₁₁ are phenyl, R₁₂ is 4-trifluoromethyl or 3,5-bis(trifluoromethyl) and n is 0.

45. Complexes according to claim 22, wherein Y is selected from
{(R)-1-[(S)-2-(bis(4-trifluoromethylphenyl)-phosphino)ferrocenyl]}ethyl bis(3,5-dimethylphenyl)phosphine,
{(R)-1-[(S)-2-(bis(4-trifluoromethylphenyl)-phosphino)ferrocenyl]}ethyl-dicyclohexylphosphine,
{(R)-1-[(S)-2-(bis(3,5-bis-(trifluoromethyl)phenyl)phosphino)ferrocenyl]}ethyl bis(3,5-dimethylphenyl)phosphine,
{(R)-1-[(S)-2-(bis(3,5-bis-(trifluoromethyl)phenyl)phosphino)ferrocenyl]}ethyl-diphenylphosphine or
{(R)-1-[(S)-2-(bis(4-trifluoromethylphenyl)phosphino)ferrocenyl]}ethyl-diphenylphosphine.

46. Use of the complexes of formulae V and VI according to claim 22 as homogeneous catalysts for the asymmetric hydrogenation of prochiral compounds containing carbon double bonds or carbon/hetero atom double bonds.

47. Use according to claim 46 for the hydrogenation of unsymmetric carbon double bonds, ketimines and ketones.

48. Use according to claim 46 for the hydrogenation of unsymmetric carbon double bonds with the rhodium complexes of formulae V and VI.

49. Process for the asymmetric hydrogenation of compounds containing carbon double bonds or carbon/hetero atom double bonds under homogeneous reaction conditions, which comprises hydrogenating said compounds in the temperature range from -20 to +80°C and under a hydrogen pressure of 10⁴ to 10⁷ Pa in the presence of catalytic amounts of a complex of formulae V or VI according to claim 22.

50. Process according to claim 49, wherein the hydrogen pressure is from 10⁵ to 6·10⁶ Pa.

51. Process according to claim 49, wherein the temperature is in the range from -10 to +50°C.

52. Process according to claim 49, wherein the amount of catalyst is chosen such that the molar ratio of compound to be hydrogenated (substrate) to complex of formula V or VI is 100,000 to 20.

53. Process according to claim 49, wherein the reaction is carried out in the presence of a solvent.

54. Process according to claim 49, wherein the solvent is methanol or a mixture of methanol and benzene or toluene.

## Revendications

1. Les composés de formule I 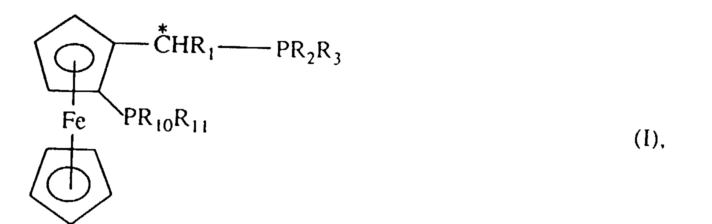 où R₁ signifie un groupe C₁-C₈-alkyle, phényle ou phényle substitué par 1 à 3 groupes C₁-C₄-alkyle ou C₁-C₄-alcoxy; R₂ signifie un reste de formule II 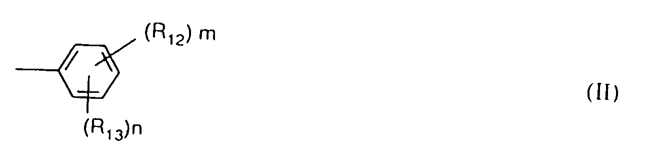 où R₁₂ signifie un groupe C₁-C₅-alkyle partiellement ou complètement substitué par le fluor;
R₁₃ signifie un groupe C₁-C₄-alkyle, C₁-C₄-alcoxy, -SiR₄R₅R₆, un halogène, -SO₃M, -CO₂M, -PO₃M, -NR₇R₈ ou -[⊕NR₇R₈R₉]X^{⊖};
m signifie un nombre de 1 à 3, n signifie 0 ou un nombre de 1 à 4 et la somme de m+n est de 1 à 5;
R₃, R₁₀, R₁₁ indépendamment les uns des autres ont la même signification que R₂ ou représentent indépendamment les uns des autres un groupe C₁-C₁₂-alkyie, C₅-C₁₂-cycloalkyle, phényle, C₅-C₁₂-cycloalkyle, substitué par un groupe C₁-C₄-alkyle ou C₁-C₄-alcoxy ou phényle substitué par 1 à 3 groupes C₁-C₄-alkyle, C₁-C₄-alcoxy, -SiR₄R₅R₆, un halogène, -SO₃M, -CO₂M, -PO₃M, -NR₇R₈ ou -[⊕NR₇R₈R₉]X^{⊖};
R₄, R₅ et R₆ signifient indépendamment les uns des autres un groupe C₁-C₁₂-alkyle ou phényle;
R₇ et R₈ représentent H, un groupe C₁-C₁₂-alkyle, phényle ou bien
R₇ et R₈ représentent ensemble un groupe tétraméthylène, pentaméthylène ou 3-oxa-1,5-pentylène;
R₉ signifie H ou un groupe C₁-C₄-alkyle;
M signifie H ou un métal alcalin, X^{⊖} signifie l'anion d'un acide monobasique et * caractérise un atome de carbone stéréogène, sous forme de leurs racémates et de leurs diastéréomères ou de mélanges de diastéréomères, à l'exception de la {(R)-1-[(S)-2-diphénylphosphino)ferrocényl]}éthyl-di-(4-trifluorométhylphényl)phosphine.

2. Les composés de formule I selon la revendication 1, caractérisés en ce que R₁ en tant que groupe alkyle est linéaire.

3. Les composés de formule I selon la revendication 2, caractérisés en ce que R₁ représente un groupe C₁-C₄-alkyle.

4. Les composés de formule I selon la revendication 3, caractérisés en ce que R₁ signifie un groupe méthyle ou éthyle.

5. Les composés de formule I selon la revendication 1, caractérisés en ce que R₁ représente un groupe phényle ou un groupe phényle substitué par 1 ou 2 groupes C₁-C₄-alkyle ou C₁-C₄-alcoxy.

6. Les composés de formule I selon la revendication 1, caractérisés en ce que R₃, R₁₀ et R₁₁ représentent un groupe C₁-C₈-alkyle.

7. Les composés de formule I selon la revendication 1, caractérisés en ce que R₃, R₁₀ et R₁₁ en tant que groupe cycloalkyle, contiennent de 5 à 8 atomes de carbone dans le cycle.

8. Les composés de formule I selon la revendication 7, caractérisés en ce que R₃, R₁₀ et R₁₁ en tant que groupe cycloalkyle, représentent un groupe cyclopentyle ou cyclohexyle.

9. Les composés de formule I selon la revendication 1, caractérisés en ce que R₃, R₁₀ et R₁₁ représentent un groupe phényle, 2-méthyl-, 3-méthyl-, 4-méthyl-, 2-ou 4-éthyl-, 2- ou 4-iso-propyl-, 2- ou 4-tert.-butyl-, 2-méthoxy-, 3-méthoxy-, 4-méthoxy-, 2- ou 4-éthoxy-, 4-triméthylsilyl-, 2- ou 4-fluoro-, 2,4-difluoro-, 2- ou 4-chloro-, 2,4-dichloro-, 2,4-diméthyl-, 3,5-diméthyl-, 2-méthoxy-4-méthyl-, 3,5-diméthyl-4-méthoxy-, 3,5-diméthyl-4-(diméthylamino)-, 2- ou 4-amino-, 2- ou 4-méthylamino-, 2- ou 4-(diméthylamino)-, 2- ou 4-SO₃H-, 2- ou 4-SO₃Na-, 2- ou 4-[⊕NH₃Cl^{⊖}]-, 2,4,6-triméthyl-phen-1-yle ou 3,4,5-triméthylphen-1-yle.

10. Les composés de formule I selon la revendication 1, caractérisés en ce que R₁₂ signifie un groupe C₁-C₃-perfluoroalkyle.

11. Les composés de formule I selon la revendication 1, caractérisés en ce que R₁₃ signifie un groupe C₁-C₄-alkyle ou C₁-C₄-alcoxy.

12. Les composés de formule I selon la revendication 1, caractérisés en ce que R₁₂ signifie un groupe 4-trifluorométhyle ou 3,5-di-(trifluorométhyle).

13. Les composés de formule I selon la revendication 1, caractérisés en ce que R₃, R₁₀ et R₁₁ représentent des restes identiques et signifient un groupe phényle, cyclohexyle, tert.-butyle, 2- ou 4-méthylphen-1-yle, 2- ou 4-méthoxyphen-1-yle, 2- ou 4-(diméthylamino)phen-1-yle, 3,5-di-méthyl-4-(diméthylamino)phen-1-yle ou 3,5-diméthyl-4-méthoxyphen-1-yle.

14. Les composés de formule I selon la revendication 1, caractérisés en ce que R₂ et R₃ sont identiques et représentent un reste de formule II et R₁₀ et R₁₁ représentent des restes identiques et signifient un groupe phényle, cylohexyle, tert.-butyle, 2- ou 4-méthylphen-1-yle, 2- ou 4-méthoxyphen-1-yle, 2- ou 4-(diméthylamino)phen-1-yle, 3,5-diméthyl-4-(diméthylamino)phen-1-yle ou 3,5-diméthyl-4-méthoxyphen-1-yle.

15. Les composés de formule I selon la revendication 1, caractérisés en ce que R₂ et R₃ sont identiques et représentent un reste de formule II, R₁₀ signifie un groupe phényle et R₁₁ signifie un groupe cyclohexyle, 2- ou 4-méthylphen-1-yle, 2- ou 4-méthoxyphen-1-yle, 4-(diméthylamino)phen-1-yle, 3,5-diméthyl-4-(diméthylamino)phen-1-yle, 3,5-diméthyl-4-méthoxyphen- 1-yle ou 4-tert.-butyl-phen-1-yle.

16. Les composés de formule I selon la revendication 1, caractérisés en ce que R₁ signifie un groupe méthyle, R₂ et R₃ sont identiques et représentent un reste de formule II, R₁₀ et R₁₁ représentent des restes identiques et signifient un groupe phényle ou cyclohexyle.

17. Les composés de formule I selon la revendication 1, caractérisés en ce que R₂, R₃, R₁₀ et R₁₁ sont identiques et signifient un reste de formule II.

18. Les composés de formule I selon la revendication 1, caractérisés en ce que R₁ signifie un groupe méthyle, R₂, R₃, R₁₀ et R₁₁ sont identiques et représentent un reste de formule II, R₁₂ signifie un groupe 4-trifluorométhyle ou 3,5-di(trifluorométhyle) et n signifie 0.

19. Les composés de formule I selon la revendication 1, caractérisés en ce que R₁ signifie un groupe méthyle, R₂ et R₃ sont identiques et représentent un reste de formule II, R₁₀ et R₁₁ signifient un groupe phényle, R₁₂ signifie un groupe 4-trifluorométhyle ou 3,5-di(trifluorométhyle) et n signifie 0.

20. Les composés de formule I selon la revendication 1, caractérisés en ce qu'il s'agit de
la {(R)-1-[(S)-2-(di-(4-trifluorométhylphényl)-phosphino)ferrocényl]}éthyl-di(3,5-diméthylphényl)phosphine,
la {(R)-1-[(S)-2-(di-(4-trifluorométhylphényl)-phosphino)ferrocényl]}éthyl-dicyclohexylphosphine,
la{(R)-1-[(S)-2-(di-(3,5-bis-(trifluorométhyl)phényl)phosphino)ferrocényl]}éthyl-di(3,5-diméthylphényl)phosphine,
la {(R)-1-[(S)-2-(di-(3,5-bis-(trifluorométhyl)phényl)phosphino)ferrocényl]}éthyldiphénylphosphine ou bien
la {(R)-1-[(S)-2-(di-(4-trifluorométhylphényl)phosphino)ferrocényl]}éthyl-diphénylphosphine.

21. Un procédé de préparation des composés de formule I selon la revendication 1, caractérisé en ce qu'on fait réagir soit un composé de formule III 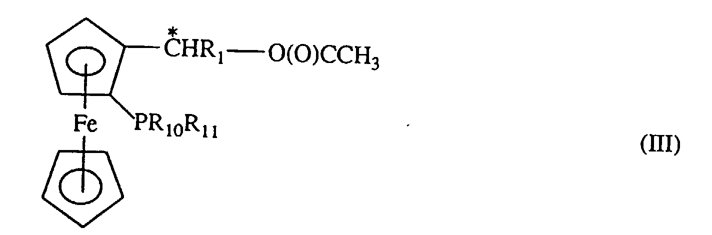 en présence d'un solvant inerte, à la température ambiante ou à une température élevée, avec une phosphine de formule IV
HPR₂R₃ (IV)
ou bien on fait réagir un composé de formule VII 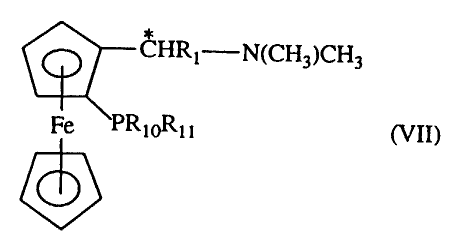 où R₁, R₁₀ et R₁₁ ont les significations indiquées sous la formule I, en présence d'un solvant inerte, à la température ambiante ou à une température élevée, avec une phosphine de formule IV
HPR₂R₃ (IV)
R₁, R₂, R₃, R₁₀ et R₁₁ ayant les significations indiquées à la revendication 1.

22. Les complexes des formules V et VI
[X₁M₁YZ] (V),
[X₁M₁Y]^{⊕}A₁^{⊖} (VI),
où X₁ signifie deux C₂-C₁₂-oléfines ou un C₅-C₁₂-diène, Z signifie CI, Br ou I, A₁^{⊖} signifie l'anion d'un oxyacide ou d'un complexe acide, M₁ signifie Rh ou Ir et Y représente un composé de formule I selon la revendication 1.

23. Les complexes selon la revendication 22, caractérisés en ce qu'il s'agit de ceux de formule VI.

24. Les complexes selon la revendication 22, caractérisés en ce que X₁ représente 2 groupes éthylène, 1,5-hexadiène, 1,5-cyclooctadiène ou norbornadiène.

25. Les complexes selon la revendication 22, caractérisés en ce que Z signifie Ci ou Br.

26. Les complexes selon la revendication 22, caractérisés en ce que Z signifie ClO₄^{⊖}, FSO₃^{⊖}, CH₃SO₃^{⊖}, CF₃SO₃^{⊖} , BF₄^{⊖}, PF₆^{⊖}, SbCl₆^{⊖}, AsF₆^{⊖} et SbF₆^{⊖} .

27. Les complexes selon la revendication 22, caractérisés en ce que R₁ en tant que groupe alkyle est linéaire.

28. Les complexes selon la revendication 27, caractérisés en ce que R₁ représente un groupe C₁-C₄-alkyle.

29. Les complexes selon la revendication 28, caractérisés en ce que R₁ signifie un groupe méthyle ou éthyle.

30. Les complexes selon la revendication 22, caractérisés en ce que R₁ représente un groupe phényle ou un groupe phényle substitué par 1 ou 2 groupes C₁-C₄-alkyle ou C₁-C₄-alcoxy.

31. Les complexes selon la revendication 22, caractérisés en ce que R₃, R₁₀ et R₁₁ représentent un groupe C₁-C₈-alkyle.

32. Les complexes selon la revendication 22, caractérisés en ce que R₃, R₁₀ et R₁₁ en tant que groupe cycloalkyle contiennent de 5 à 8 atomes de carbone dans le cycle.

33. Les complexes selon la revendication 32, caractérisés en ce que R₃, R₁₀ et R₁₁ en tant que groupe cycloalkyle représentent un groupe cyclopentyle ou cyclohexyle.

34. Les complexes selon la revendication 22, caractérisés en ce que R₃, R₁₀ et R₁₁ représentent un groupe phényle, 2-méthyl-, 3-méthyl-, 4-méthyl-, 2- ou 4-éthyl-, 2- ou 4-iso-propyl-, 2- ou 4-tert.-butyl-, 2-méthoxy-, 3-méthoxy-, 4-méthoxy-, 2- ou 4-éthoxy-, 4-triméthylsilyl-, 2- ou 4-fluoro-, 2,4-difluoro-, 2- ou 4-chloro-, 2,4-dichloro-, 2,4-diméthyl-, 3,5-diméthyl-, 2-méthoxy-4-méthyl-, 3,5-diméthyl-4-méthoxy-, 3,5-diméthyl-4-(diméthylamino)-, 2- ou 4-amino-, 2- ou 4-méthylamino-, 2- ou 4-(diméthylamino)-, 2- ou 4-SO₃H-, 2- ou 4-SO₃Na-, 2- ou 4-[⊕NH₃Cl^{⊖}]-, 2,4,6-triméthylphen- 1-yle ou 3,4,5-triméthylphen-1-yle.

35. Les complexes selon la revendication 22, caractérisés en ce que R₁₂ signifie un groupe C₁-C₃perfluoroalkyle.

36. Les complexes selon la revendication 22, caractérisés en ce que R₁₃ signifie un groupe C₁-C₄alkyle, C₁-C₄alcoxy.

37. Les complexes selon la revendication 22, caractérisés en ce que R₁₂ signifie un groupe 4-trifluorométhyle ou 3,5-di-(trifluorométhyle).

38. Les complexes selon la revendication 22, caractérisés en ce que R₃, R₁₀ et R₁₁ représentent des restes identiques et signifient un groupe phényle, cyclohexyle, tert.-butyle, 2- ou 4-méthylphen-1-yle, 2- ou 4-méthoxyphen-1-yle, 2- ou 4-(diméthylamino)phen-1-yle, 3,5-diméthyl-4-(diméthylamino)phen-1-yle ou 3,5-diméthyl-4-méthoxyphen-1-yle.

39. Les complexes selon la revendication 22, caractérisés en ce que R₂ et R₃ sont identiques et représentent un reste de formule II et R₁₀ et R₁₁ représentent des restes identiques et signifient un groupe phényle, cyclohexyle, tert.-butyle, 2-ou 4-méthylphen-1-yle, 2- ou 4-méthoxyphen-1-yle, 2- ou 4-(diméthylamino)phen-1-yle, 3,5-diméthyl-4-(diméthylamino)phen-1-yle ou 3,5-diméthyl-4-méthoxyphen-1-yle.

40. Les complexes selon la revendication 22, caractérisés en ce que R₂ et R₃ sont identiques et signifient un reste de formule II, R₁₀ signifie un groupe phényle et R₁₁ signifie un groupe cyclohexyle, 2- ou 4-méthylphen-1-yle, 2- ou 4-méthoxyphen-1-yle, 4-(diméthylamino)phen-1-yle, 3,5-diméthyl-4-(diméthylamino)phen-1-yle, 3,5-diméthyl-4-méthoxyphen-1-yle ou 4-tert.-butyl-phen-1-yle.

41. Les complexes selon la revendication 22, caractérisés en ce que R₁ signifie un groupe méthyle, R₂ et R₃ sont identiques et représentent un reste de formule II, R₁₀ et R₁₁ représentent des restes identiques et signifient un groupe phényle ou cyclohexyle.

42. Les complexes selon la revendication 22, caractérisés en ce que R₂, R₃, R₁₀ et R₁₁ sont identiques et signifient un reste de formule II.

43. Les complexes selon la revendication 22, caractérisés en ce que R₁ signifie un groupe méthyle, R₂, R₃, R₁₀ et R₁₁ sont identiques et représentent un reste de formule II, R₁₂ signifie un groupe 4-trifluorométhyle ou 3,5-di(trifluorométhyle) et n signifie 0.

44. Les complexes selon la revendication 22, caractérisés en ce que R₁ signifie un groupe méthyle, R₂ et R₃ sont identiques et représentent un reste de formule II, R₁₀ et R₁₁ signifient un groupe phényle, R₁₂ signifie un groupe 4-trifluorométhyle ou 3,5-di(trifluorométhyle) et n signifie 0.

45. Les complexes selon la revendication 22, caractérisés en ce que Y signifie
la {(R)-1-[(S)-2-(di-(4-trifluorométhylphényl)-phosphino)ferrocényl]}éthyl-di(3,5-diméthylphényl)phosphine,
la {(R)-1-[(S)-2-(di-(4-trifluorométhylphényl)-phosphino)ferrocényl]}éthyldicyclohexylphosphine,
la {(R)-1-[(S)-2-(di-(3,5-bis-(trifluorométhyl)phényl)phosphino)ferrocényl]}éthyl-di(3,5-diméthylphényl)phosphine,
la {(R)-1-[(S)-2-(di-(3,5-bis-(trifluorométhyl)phényl)phosphino)ferrocényl]}éthyldiphénylphosphine ou bien
la {(R)-1-[(S)-2-(di-(4-trifluorométhylphényl)phosphino)ferrocényl]}éthyldiphénylphosphine.

46. L'utilisation des complexes des formules V et VI selon la revendication 22, comme catalyseurs homogènes pour l'hydrogénation asymétrique de composés pro-chiraux avec des doubles liaisons de carbone ou de carbone/hétéroatome.

47. L'utilisation selon la revendication 46, pour l'hydrogénation de doubles liaisons de carbone dissymétriques, de cétimines et de cétones.

48. L'utilisation selon la revendication 46, pour l'hydrogénation de doubles liaisons de carbone dissymétriques avec des complexes du rhodium des formules V et VI.

49. Un procédé d'hydrogénation asymétrique de composés avec des doubles liaisons de carbone ou de carbone/héréroatome sous des conditions de réaction homogènes, caractérisé en ce qu'on hydrogène les composés à une température de -20 à 80°C et sous pression d'hydrogène de 10⁴ à 10⁷ Pa en présence de quantités catalytiques d'un complexe des formules V ou VI selon la revendication 22.

50. Un procédé selon la revendication 49, caractérisé en ce que la pression d'hydrogène est comprise entre 10⁵ et 6.10⁶ Pa.

51. Un procédé selon la revendication 49, caractérisé en ce que la température est comprise entre -10 et 50°C.

52. Un procédé selon la revendication 49, caractérisé en ce que la quantité du catalyseur est choisie afin que le rapport molaire du composé à hydrogéner (substrat) au complexe des formules V ou VI soit compris entre 100000 et 20.

53. Un procédé selon la revendication 49, caractérisé en ce que la réaction est effectuée en présence d'un solvant.

54. Un procédé selon la revendication 49, caractérisé en ce que le solvant est le méthanol ou un mélange de méthanol avec du benzène ou du toluène.
